# EUROPEAN PATENT APPLICATION

(11) **EP 3 159 692 A1**
(43) Date of publication of application: **26.04.2017**
(21) Application number: 16199287.0
(22) Date of filing: 30.03.2012
(51) Int. Cl.: G01N 33/49, G01N 21/64, G01N 33/48, G01N 15/14, G01N 33/50, G01N 33/569, G01N 21/47, G01N 21/51

(54) **BLOOD ANALYZER, BLOOD ANALYSIS METHOD, AND COMPUTER PROGRAM**

(30) Priority: 28.04.2011 JP 2011100810
(62) Divisional of application: 12777021.2
(71) Applicant: SYSMEX CORPORATION, Kobe-shi Hyogo 651-0073 (JP)
(72) Inventor: Yoshida, Ayumu, Hyogo, 651-0073 (JP); Kataoka, Yukiko, Hyogo, 651-0073 (JP); Tsuji, Tomohiro, Hyogo, 651-0073 (JP)
(74) Representative: Hoffmann Eitle

(57) **Abstract**

A blood analyzer, a blood analysis method, and a computer program that can distinguishably detect abnormal lymphocytes from blasts and atypical lymphocytes are provided. A blood analyzer (1) prepares a measurement sample by mixing a first reagent containing a hemolyzing agent, a second reagent containing a fluorescent dye for staining nucleic acid, and a blood specimen in a sample preparation portion (22). The hemolyzing agent does not substantially contain a cationic surfactant, but contains a nonionic surfactant. A detection portion (23) measures the measurement sample and an information processing unit (5) processes a measurement data output by the detection portion (23), so that the blood analyzer (1) can detect abnormal lymphocytes and blasts separately. The information processing unit (2) outputs an analysis result based on a detection result.

## Description

### Technical Field

The present invention relates to a blood analyzer and a blood analysis method for optically measuring a blood specimen and classifying hemocytes contained in the blood specimen, and a computer program for enabling a computer to analyze blood.

### Background Art

Five types of leukocyte consisting of lymphocytes, monocytes, basophils, eosinophils, and neutrophils are present in normal peripheral blood, and many blood cell counting apparatuses have the function of classifying leukocytes contained in a blood specimen into the five types. On the other hand, cells that are not present in normal peripheral blood appear in peripheral blood affected with diseases such as viral infectious diseases and hematopoietic organ tumors. Abnormal leukocytes that appear in peripheral blood include abnormal mononuclear leukocytes and, for example, "abnormal lymphocytes", which are neoplastic mature lymphocytes, appear as abnormal mononuclear leukocytes in peripheral blood affected with diseases such as chronic lymphatic leukemia and malignant lymphoma. On the other hand, "blasts (myeloblasts and lymphoblasts)", which are immature lymphocytes, appear as abnormal mononuclear leukocytes in peripheral blood affected with acute leukemia. Moreover, "atypical lymphocytes", which are lymphocytes activated by stimulation of antigen, appear as abnormal mononuclear leukocytes in peripheral blood affected with diseases such as viral infectious disease and drug allergy. Distinguishably detecting abnormal lymphocytes from atypical lymphocytes and blasts in peripheral blood is very useful in screening or diagnosis of diseases such as chronic lymphatic leukemia in which abnormal lymphocytes appear in peripheral blood.

Patent Document 1 and Patent Document 2 disclose distinguishably detecting abnormal leukocytes including abnormal lymphocytes from normal leukocytes using reagents for classifying leukocytes into five or four categories. Specifically, these documents disclose that a hemolyzing agent containing a cationic surfactant and a nonionic surfactant, and a stain solution containing a fluorescent dye for staining nucleic acid are used as the reagents for classifying leukocytes to develop differences of fluorescence intensity and scattered light intensity between abnormal leukocytes and normal leukocytes, and to distinguishably detect abnormal leukocytes from normal leukocytes based on the differences.

Patent Document 3 discloses distinguishably detecting myeloblasts from mature leukocytes and immature granulocytes using predetermined reagents (see FIGS. 1, 2, and 5). Specifically, this document discloses that a hemolyzing agent containing a nonionic surfactant and a solubilizing agent, and a stain solution containing a fluorescent dye for staining nucleic acid are used as the above-described reagents to develop differences of fluorescence intensity and scattered light intensity between myeloblasts, mature leukocytes and immature granulocytes, and to distinguishably detect myeloblasts from mature leukocytes and immature granulocytes based on the differences.

Patent Document 4 discloses distinguishably detecting lymphoblasts and myeloblasts from mature leukocytes and immature granulocytes using predetermined reagents (see FIGS. 13A and 13B). Specifically, this document discloses that a hemolyzing agent containing a nonionic surfactant and a solubilizing agent, and a stain solution containing a fluorescent dye for staining nucleic acid are used as the above-described reagents to distinguishably detect lymphoblasts and myeloblasts from mature leukocytes and immature granulocytes.

### Citation List

### Patent Documents

Patent Document 1: US 2009/0023129A1
Patent Document 2: US 2010/0151509A1
Patent Document 3: JP 2007-263894A
Patent Document 4: JP 2010-237147A

### Summary of the Invention

### Technical Problem

However, in the method disclosed in Patent Document 1 described above, abnormal lymphocytes and blasts appear in the same area in a scattergram of side fluorescence intensity and side scattered light intensity, and therefore cannot be distinguished from each other (see FIG. 2). Furthermore, Patent Document 1 describes nothing about an appearance of atypical lymphocytes.

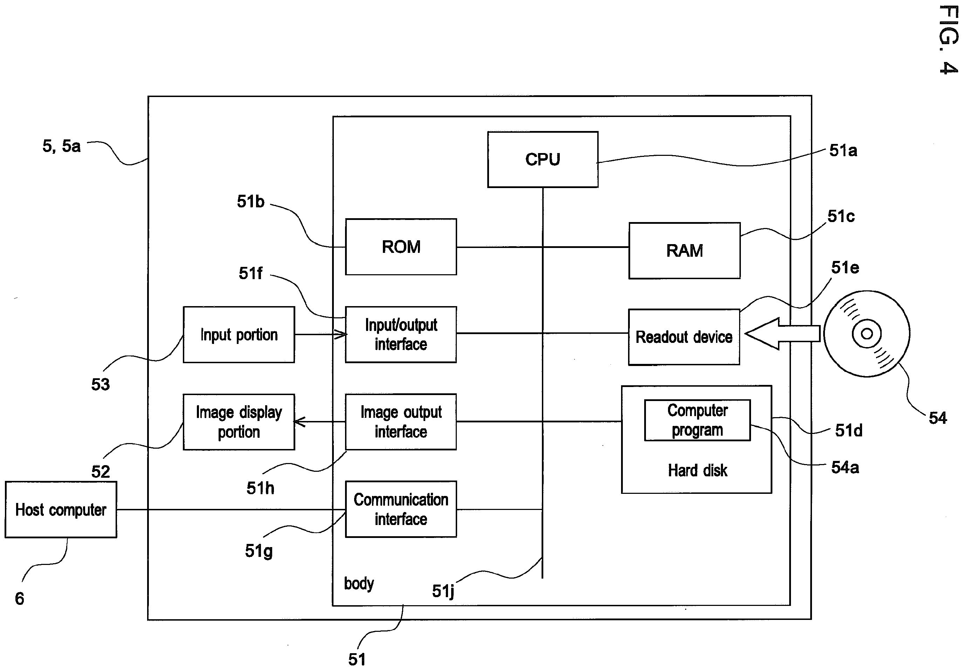

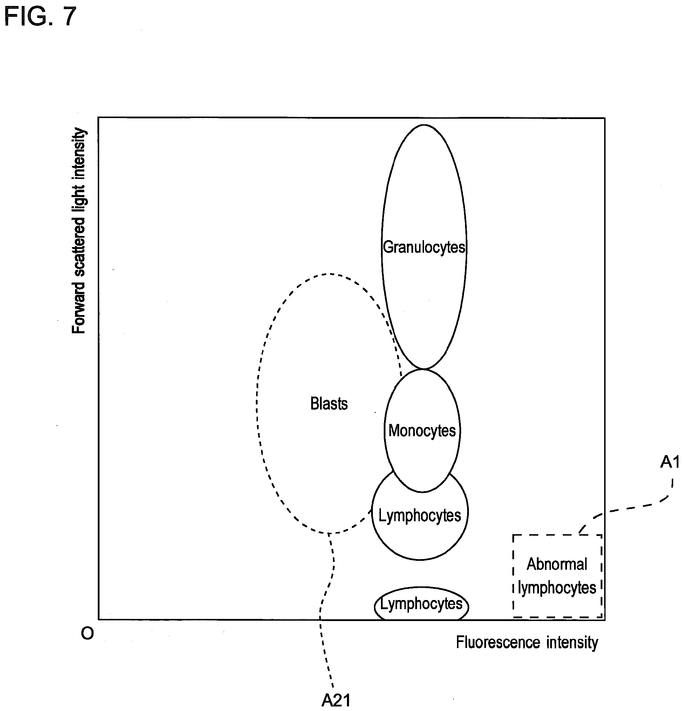

Patent Document 2 shows the area in which abnormal lymphocytes appear in a scattergram of side fluorescence intensity and side scattered light intensity (see FIG. 4 and FIG. 7), but describes nothing about an appearance of blasts and atypical lymphocytes. Furthermore, since the reagents described in Patent Document 2 have similar characteristics to the reagents described in Patent Document 1 and, in addition, the area in which abnormal lymphocytes appear in a scattergram shown in Patent Document 2 is in the same position as the area in which abnormal lymphocytes and blasts appear in a scattergram shown in Patent Document 1, it is assumed that, even in Patent Document 2, blasts also appear in the area described above in which abnormal lymphocytes appear, and blasts and abnormal lymphocytes cannot be distinguished from each other.

Furthermore, neither Patent Document 3 nor Patent Document 4 describes distinguishably detecting abnormal lymphocytes from other hemocytes.

The present invention was made in view of the foregoing circumstances, and it is a main object thereof to provide a blood analyzer, a blood analysis method, and a computer program that can distinguishably detect abnormal lymphocytes from blasts and atypical lymphocytes.

### Solution to Problem

To solve the above-described problems, a blood analyzer of one aspect of the present invention includes: a sample preparation portion for preparing a measurement sample by mixing a hemolyzing agent not substantially containing a cationic surfactant, but containing a nonionic surfactant, a blood specimen, and a fluorescent dye for staining nucleic acid; a light source for irradiating the measurement sample prepared by the sample preparation portion with light; a light-receiving portion for receiving fluorescence and scattered light that are produced by cells in the measurement sample when the light source irradiates the measurement sample with light to output a fluorescence signal relating to the received fluorescence and a scattered light signal relating to the received scattered light; a cell analysis portion for detecting cells showing fluorescence intensity and scattered light intensity in a predetermined range as abnormal lymphocytes based on the fluorescence signal and the scattered light signal output by the light-receiving portion; and an output portion for performing output based on a result of a detection made by the cell analysis portion.

In this aspect, the cell analysis portion may be configured to detect the cells showing fluorescence intensity and scattered light intensity in the predetermined range as abnormal lymphocytes distinguishably from blasts and atypical lymphocytes.

In this aspect, the light-receiving portion may be configured to receive forward scattered light and side scattered light produced by cells in the measurement sample when the measurement sample is irradiated with light to output the scattered light signal containing a forward scattered light signal relating to the received forward scattered light and a side scattered light signal relating to the received side scattered light, and the cell analysis portion may be configured to detect cells showing fluorescence intensity and scattered light intensity in a predetermined range as abnormal lymphocytes based on the fluorescence signal and at least one of the forward scattering light signal and the side scattering light signal.

In the above-described aspect, the light-receiving portion may be configured to receive forward scattered light and side scattered light produced by cells in the measurement sample when the measurement sample is irradiated with light to output the scattered light signal containing a forward scattered light signal relating to the received forward scattered light and a side scattered light signal relating to the received side scattered light, and the cell analysis portion may be configured to detect blasts distinguishably from abnormal lymphocytes and atypical lymphocytes based on at least two signals selected from the forward scattered light signal, the side scattered light signal, and the fluorescence signal.

In the above-described aspect, the cell analysis portion may be configured to detect cells showing forward scattered light intensity and side scattered light intensity in a predetermined range as blasts distinguishably from abnormal lymphocytes and atypical lymphocytes based on the forward scattered light signal and the side scattered light signal.

In the above-described aspect, the sample preparation portion may be configured to prepare a measurement sample by mixing the hemolyzing agent not substantially containing a cationic surfactant, but containing a nonionic surfactant and an anionic surfactant, the blood specimen, and the fluorescent dye.

In the above-described aspect, the sample preparation portion may be configured to prepare a second measurement sample by mixing a second hemolyzing agent for measuring nucleated erythrocytes, a blood specimen, and a second fluorescent dye for measuring nucleated erythrocytes, the light-receiving portion may be configured to receive fluorescence and scattered light produced by cells in the second measurement sample when the light source irradiates the second measurement sample with light to output a second fluorescence signal relating to the received fluorescence and a second scattered light signal relating to the received scattered light, and the cell analysis portion may be configured to perform a detection process for detecting nucleated erythrocytes in the second measurement sample based on the second fluorescence signal and the second scattered light signal output by the light-receiving portion, and detect the cells showing fluorescence intensity and scattered light intensity in the predetermined range as abnormal lymphocytes if a result of the detection process satisfies a predetermined condition.

A blood analyzer of another aspect of the present invention includes: a sample preparation portion for preparing a first measurement sample from a blood specimen and a first reagent, and a second measurement sample from a blood specimen and a second reagent; a light source for irradiating the first measurement sample and the second measurement sample prepared by the sample preparation portion with light; a light-receiving portion for receiving fluorescence and scattered light that are produced by cells in the first measurement sample when the light source irradiates the first measurement sample with light to output a first fluorescence signal relating to the received fluorescence and a first scattered light signal relating to the received scattered light, and receiving fluorescence and scattered light that are produced by cells in the second measurement sample when the light source irradiates the first measurement sample with light to output a second fluorescence signal relating to the received fluorescence and a second scattered light signal relating to the received scattered light; a cell analysis portion for performing a detection process for detecting nucleated erythrocytes in the second measurement sample based on the second fluorescence signal and the second scattered light signal, and detecting cells showing fluorescence intensity and scattered light intensity in a predetermined range as abnormal lymphocytes based on the first fluorescence signal and the first scattered light signal if a result of the detection process satisfies a predetermined condition; and an output portion for performing output based on a result of a detection made by the cell analysis portion.

In the above-described aspect, the cell analysis portion may be configured to perform an obtaining process, as the detection process, for obtaining values based on the number of cells showing fluorescence intensity and scattered light intensity in a second predetermined range on the basis of the second fluorescence signal and the second scattered light signal, and detect the cells showing fluorescence intensity and scattered light intensity in the predetermined range as abnormal lymphocytes if the obtained value is not greater than a predetermined threshold.

A blood analysis method of one aspect of the present invention includes steps of preparing a measurement sample by mixing a hemolyzing agent not substantially containing a cationic surfactant, but containing a nonionic surfactant, a blood specimen, and a fluorescent dye for staining nucleic acid; irradiating the measurement sample prepared with light; receiving fluorescence and scattered light that are produced by cells in the measurement sample when the measurement sample is irradiated with light to obtain a fluorescence signal relating to the received fluorescence and a scattered light signal relating to the received scattered light; detecting cells showing fluorescence intensity and scattered light intensity in a predetermined range as abnormal lymphocytes based on the fluorescence signal and the scattered light signal; and performing output based on a result of a detection.

In this aspect, in the step of detecting abnormal lymphocytes, the cells showing fluorescence intensity and scattered light intensity in the predetermined range may be detected as abnormal lymphocytes distinguishably from blasts and atypical lymphocytes.

In this aspect, in the step of receiving the fluorescence signal and the scattered light signal, fluorescence, forward scattered light, and side scattered light that are produced by cells in the measurement sample may be received when the measurement sample is irradiated with light to obtain the fluorescence signal relating to the received fluorescence and the scattered light signal containing a forward scattered light signal relating to the received forward scattered light and a side scattered light signal relating to the received side scattered light, and in the step of detecting abnormal lymphocytes, cells showing fluorescence intensity and scattered light intensity in a predetermined range may be detected as abnormal lymphocytes based on the fluorescence signal and at least one of the forward scattering light signal and the side scattering light signal.

In the above-described aspect, in the step of obtaining the fluorescence signal and the scattered light signal, fluorescence, forward scattered light and side scattered light that are produced by cells in the measurement sample may be received when the measurement sample is irradiated with light to obtain the fluorescence signal relating to the received fluorescence and the scattered light signal containing a forward scattered light signal relating to the received forward scattered light and a side scattered light signal relating to the received side scattered light, and the blood analysis method may further include a step of detecting blasts distinguishably from abnormal lymphocytes and atypical lymphocytes based on at least two signals selected from the forward scattered light signal, the side scattered light signal, and the fluorescence signal.

In the above-described aspect, in the step of detecting blasts, cells showing forward scattered light intensity and side scattered light intensity in a predetermined range may be detected as blasts distinguishably from abnormal lymphocytes and atypical lymphocytes based on the forward scattering light signal and the side scattering light signal.

In the above-described aspect, in the step of preparing the measurement sample, the measurement sample may be prepared by mixing the hemolyzing agent not substantially containing a cationic surfactant, but containing a nonionic surfactant and an anionic surfactant, the blood specimen, and the fluorescent dye.

A blood analysis method of another aspect of the present invention includes steps of preparing a first measurement sample from a blood specimen and a first reagent; irradiating the first measurement sample prepared with light; receiving fluorescence and scattered light that are produced by cells in the first measurement sample when the first measurement sample is irradiated with light to obtain a first fluorescence signal relating to the received fluorescence and a first scattered light signal relating to the received scattered light; preparing a second measurement sample from a blood specimen and a second reagent; irradiating the second measurement sample prepared with light; receiving fluorescence and scattered light that are produced by cells in the second measurement sample when light is irradiated onto the second measurement sample to obtain a second fluorescence signal relating to the received fluorescence and a second scattered light signal relating to the received scattered light; performing a detection process for detecting nucleated erythrocytes in the second measurement sample based on the second fluorescence signal and the second scattered light signal, and determining whether a result of the detection process satisfies a predetermined condition; detecting cells showing fluorescence intensity and scattered light intensity in a predetermined range as abnormal lymphocytes based on the first fluorescence signal and the first scattered light signal if the result of the detection process satisfies the predetermined condition; and performing output based on a result of a detection.

A computer program of one aspect of the present invention enables a computer including a communication portion and an output portion to analyze a blood specimen, and the computer program enables the computer to perform steps of the communication portion receiving a fluorescence signal and a scattered light signal relating to fluorescence and scattered light that are produced by cells in a measurement sample prepared by mixing a hemolyzing agent not substantially containing a cationic surfactant, but containing a nonionic surfactant, a blood specimen, and a fluorescence dye for staining nucleic acid when the measurement sample is irradiated with light; detecting cells showing fluorescence intensity and scattered light intensity in a predetermined range as abnormal lymphocytes based on the fluorescence signal and the scattered light signal; and enabling the output portion to produce an output based on a result of a detection.

In this aspect, in the above-described step of detecting the abnormal lymphocytes, the computer program may enable the computer to detect the cells showing fluorescence intensity and scattered light intensity in the predetermined range as abnormal lymphocytes distinguishably from blasts and atypical lymphocytes.

A computer program of another aspect of the present invention enables a computer including a communication portion and an output portion to analyze a blood specimen, and the computer program enables the computer to perform steps of the communication portion receiving a first fluorescence signal and a first scattered light signal relating to fluorescence and scattered light that are produced by cells in a first measurement sample prepared from a blood specimen and a first reagent when the first measurement sample is irradiated with light, and a second fluorescence signal and a second scattered light signal relating to fluorescence and scattered light that are produced by cells in a second measurement sample prepared from a blood specimen and a second reagent when the second measurement sample is irradiated with light; performing a detection process for detecting nucleated erythrocytes in the second measurement sample based on the second fluorescence signal and the second scattered light signal, and determining whether a result of the detection process satisfies a predetermined condition; detecting cells showing fluorescence intensity and scattered light intensity in a predetermined range as abnormal lymphocytes based on the first fluorescence signal and the first scattered light signal if the result of the detection process satisfies the predetermined condition; and enabling the output portion to perform output based on a result of a detection.

### Advantageous Effects of the Invention

With a blood analyzer, a blood analysis method, and a computer program according to the present invention, it is possible to distinguishably detect abnormal lymphocytes from blasts and atypical lymphocytes.

### Brief Description of Drawings

FIG. 1 is a perspective view showing an external appearance of a blood analyzer according to Embodiment 1.
FIG. 2 is a block diagram showing the configuration of a measurement unit according to Embodiment 1.
FIG. 3 is a schematic diagram showing the configuration outline of an optical detector.
FIG. 4 is a block diagram showing the configuration of an information processing unit according to Embodiment 1.
FIG. 5 is a flowchart illustrating the procedure of operation in a measurement step performed by the blood analyzer according to Embodiment 1.
FIG. 6 is a flowchart illustrating the procedure of processing in a data processing step performed by the blood analyzer according to Embodiment 1.
FIG. 7 is a schematic diagram showing a scattergram of forward scattered light intensity and fluorescence intensity in measurement data.
FIG. 8 is a schematic diagram showing a scattergram of fluorescence intensity and side scattered light intensity in measurement data.
FIG. 9 is a schematic diagram showing a scattergram of forward scattered light intensity and side scattered light intensity in measurement data.
FIG. 10A shows examples of scattergrams obtained when measuring a blood specimen containing myeloblasts.
FIG. 10B shows examples of scattergrams obtained when measuring a blood specimen containing lymphoblasts.
FIG. 10C shows examples of scattergrams obtained when measuring a blood specimen containing abnormal lymphocytes.
FIG. 10D shows examples of scattergrams obtained when measuring a blood specimen containing atypical lymphocytes.
FIG. 10E shows examples of scattergrams obtained when measuring a normal blood specimen.
FIG. 11 is a diagram showing an example of an analysis result screen of the blood analyzer.
FIG. 12 is a block diagram showing the configuration of a measurement unit according to Embodiment 2.
FIG. 13 is a flowchart illustrating the procedure of operation in a second measurement step performed by the blood analyzer according to Embodiment 2.
FIG. 14 is a flowchart illustrating the procedure of processing in a data processing step performed by the blood analyzer according to Embodiment 2.
FIG. 15 is a schematic diagram showing a scattergram of forward scattered light intensity and fluorescence intensity in second measurement data.
FIG. 16A shows examples of scattergrams obtained when measuring a blood specimen containing abnormal lymphocytes.
FIG. 16B shows examples of scattergrams obtained when measuring a blood specimen containing nucleated erythrocytes.

### Description of Embodiments

Hereinafter, preferred embodiments of the present invention will be described with reference to the accompanying drawings.

### Embodiment 1

### Configuration of Blood Analyzer

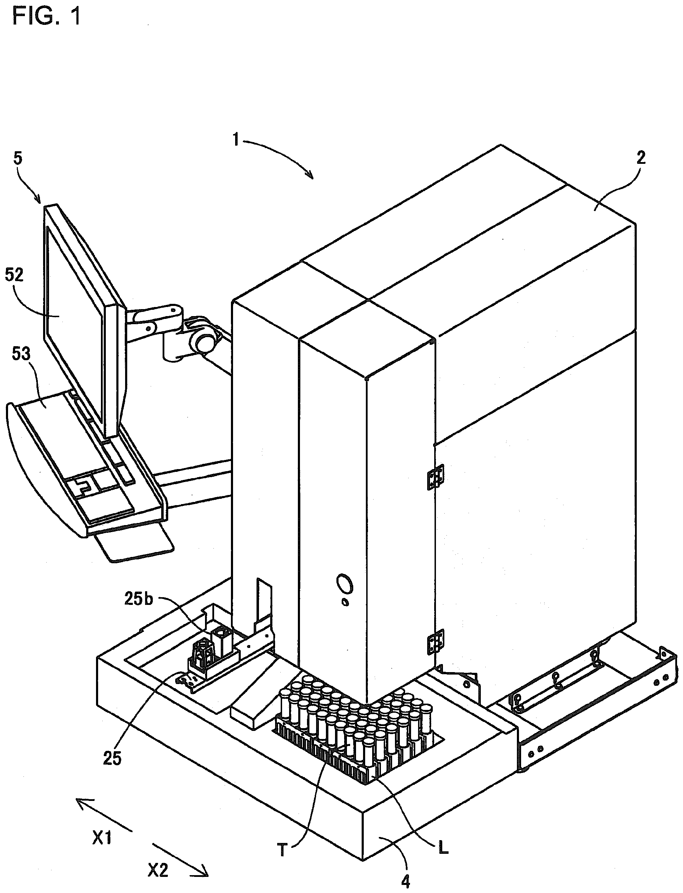

FIG. 1 is a perspective view showing an external appearance of a blood analyzer according to this embodiment. A blood analyzer 1 according to this embodiment is a multiple-item hemocyte analyzer for detecting leukocytes, erythrocytes, platelets, and the like, which are hemocytes contained in a blood specimen, and counting each type of the hemocyte. As shown in FIG. 1, the blood analyzer 1 includes a measurement unit 2, a specimen carrying unit 4 disposed on the front side of the measurement unit 2, and an information processing unit 5 that can control the measurement unit 2 and the specimen carrying unit 4.

A blood specimen that is peripheral blood collected from a patient is housed in a specimen container (blood collecting tube). A plurality of specimen containers are held in a sample rack, and the sample rack is carried by the specimen carrying unit 4, and thereby the blood specimen is supplied to the measurement unit 2.

### Configuration of Measurement Unit

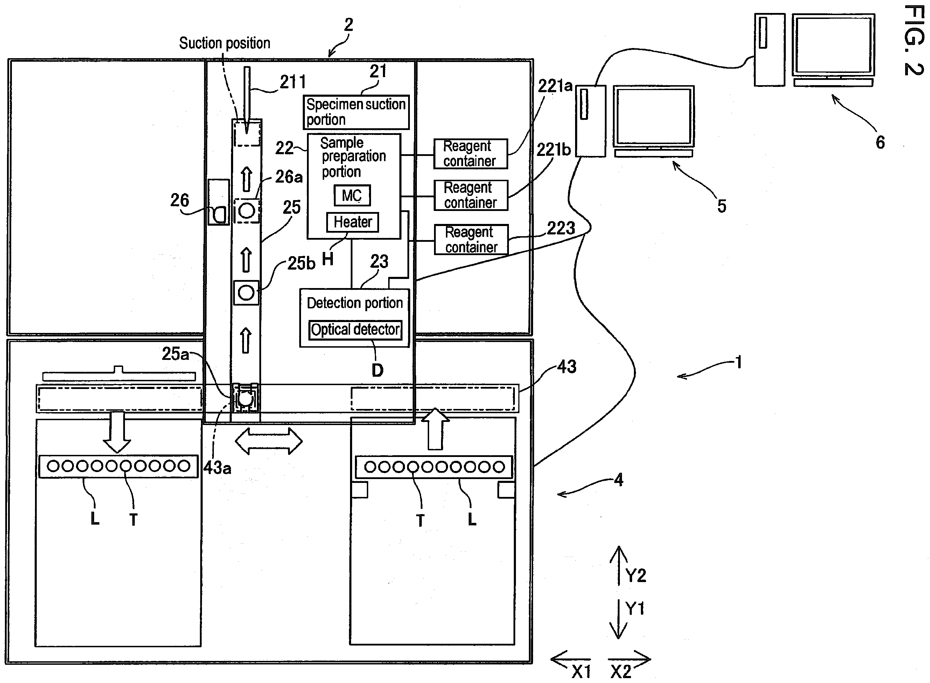

The configuration of the measurement unit will be described. FIG. 2 is a block diagram showing the configuration of the measurement unit. As shown in FIG. 2, the measurement unit 2 includes a specimen suction portion 21 that suctions blood as a specimen from the specimen container (blood collecting tube) T, a sample preparation portion 22 that prepares a measurement sample used for measurement from the blood suctioned by the specimen suction portion 21, and a detection portion 23 that detects hemocytes in the measurement sample prepared by the sample preparation portion 22. The measurement unit 2 further includes an inlet (see FIG. 1) for taking, into the measurement unit 2, the specimen container T housed in the sample rack L carried by a rack carrying portion 43 of the specimen carrying unit 4, and a specimen container carrying portion 25 that takes the specimen container T from the sample rack L into the measurement unit 2 and carries the specimen container T to a suction position where blood is suctioned by the specimen suction portion 21.

As shown in FIG. 2, the specimen suction portion 21 includes a suction tube 211. The specimen suction portion 21 also includes a syringe pump. Furthermore, the suction tube 211 is vertically movable, and is configured to suction the blood contained in the specimen container T that has been carried to the suction position when moved downward.

The sample preparation portion 22 includes a mixing chamber MC. The suction tube 211 suctions a predetermined amount of a whole blood specimen from the specimen container T using the syringe pump. The specimen thus suctioned is transferred to the position of the mixing chamber MC, and a predetermined amount of the whole blood specimen is dispensed to the mixing chamber MC using the syringe pump. The sample preparation portion 22 also includes a heater H for heating the mixing chamber MC.

The sample preparation portion 22 is connected via a tube with a reagent container 221a for housing a first reagent, a reagent container 221b for housing a second reagent, and a reagent container 223 for housing a sheath fluid (diluting fluid). The sample preparation portion 22 is also connected with a compressor, and the respective reagents can be drawn from the corresponding reagent containers 221a, 221b, and 223 with the pressure generated by the compressor.

The first reagent is a hemolyzing agent for distinguishably detecting abnormal lymphocytes, atypical lymphocytes and blasts. The hemolyzing agent that contains a nonionic surfactant and substantially no cationic surfactant can be used as the first reagent. Use of the hemolyzing agent allows erythrocytes to be hemolyzed and the cell membranes of normal leukocytes and abnormal mononuclear leukocytes (atypical lymphocytes, abnormal lymphocytes, and blasts) to be damaged. Accordingly, normal leukocytes and abnormal mononuclear leukocytes are more likely to be stained with a fluorescent dye that will be described below.

An "abnormal lymphocyte" refers to a neoplastic mature lymphocyte. The abnormal lymphocytes appear in peripheral blood of patients who have a disease such as chronic lymphatic leukemia and malignant lymphoma. Furthermore, an "atypical lymphocyte" refers to a lymphocyte that is activated by stimulation of antigen and changes its form in response to the stimulation. The atypical lymphocytes appear in peripheral blood of patients who have a disease such as viral infectious disease and drug allergy.

A "blast" refers to an immature lymphocyte such as a myeloblast and a lymphoblast. The myeloblasts appear in peripheral blood of patients who have acute myelocytic leukemia, and the lymphoblasts appear in peripheral blood of patients who have acute lymphocytic leukemia.

Here, it is preferable that the nonionic surfactant is a polyoxyethylene-based nonionic surfactant. Specific examples of polyoxyethylene-based nonionic surfactants include those represented by structural formula (I) below:

R₁-R₂- (CH₂CH₂O)n-H (I)

where R₁ is an alkyl, alkenyl, or alkynyl group having 9 to 25 carbon atoms; R₂ is -O-, -COO-, or: 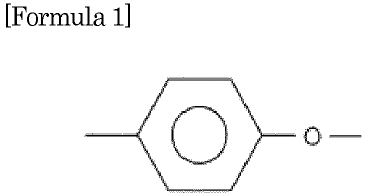 and n is an integer of 10 to 40.

Specific examples of surfactants represented by structural formula (I) above include polyoxyethylene (15) oleyl ether, polyoxyethylene (15) cetyl ether, polyoxyethylene (16) oleyl ether, polyoxyethylene (20) oleyl ether, polyoxyethylene (20) lauryl ether, polyoxyethylene (20) stearyl ether, and polyoxyethylene (20) cetyl ether, with polyoxyethylene (20) oleyl ether being preferable. The first reagent may contain one or two or more surfactants.

The concentration of surfactant contained in the first reagent can be suitably selected according to the kind of surfactant, the osmotic pressure of the hemolyzing agent, and like factors. For example, when the surfactant is polyoxyethylene oleyl ether, the concentration of surfactant contained in the first reagent is 0.5 to 50.0 g/L and preferably 1.0 to 20.0 g/L.

The first reagent may contain in addition to the nonionic surfactant a solubilizing agent to sufficiently shrink the hemolyzed erythrocytes so that the hemolyzed erythrocytes form a ghost population that does not adversely affect measurement. The solubilizing agent is used to assist an action for hemolyzing the erythrocytes due to the nonionic surfactants. Anionic surfactants can be used as solubilizing agents that may be contained in the first reagent, and examples thereof include sarcosine derivatives, cholic acid derivatives, methylglucanamide, n-octyl-ß-glucoside, sucrose monocaprate, and N-formylmethylleucylalanine, with sarcosine derivatives being particularly preferable. The first reagent may contain one or two or more solubilizing agents.

Examples of sarcosine derivatives include compounds represented by structural formula (II) below or salts thereof. 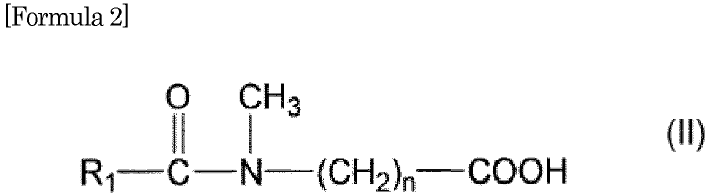 where R₁ is a C10-22 alkyl group and n is 1 to 5. Specific examples of sarcosine derivatives include sodium N-lauroylsarcosinate, sodium lauroyl methyl ß-alanine, and lauroyl sarcosine, with sodium N-lauroylsarcosinate being particularly preferable.

Examples of cholic acid derivatives include compounds represented by structural formula (III) below or salts thereof. 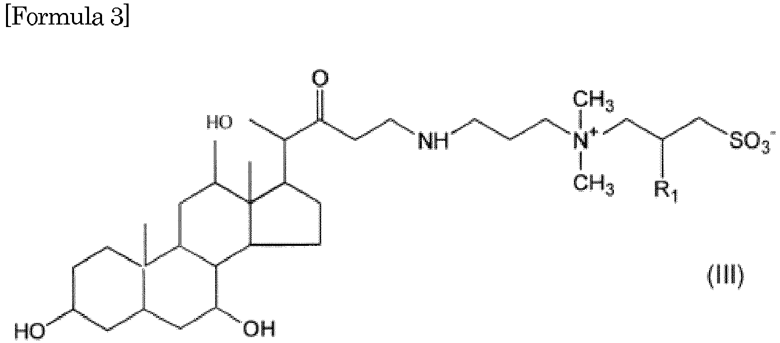 where R₁ is a hydrogen atom or a hydroxyl group. Specific examples of cholic acid derivatives include CHAPS (3-[(3-cholamidopropyl)dimethylammonio]-1-propanesulfonate) and CHAPSO ([(3-cholamidopropyl)dimethylammonio]-2-hydroxy-1-propanesulfonate).

Examples of methylglucanamides include compounds represented by structural formula (IV) below: 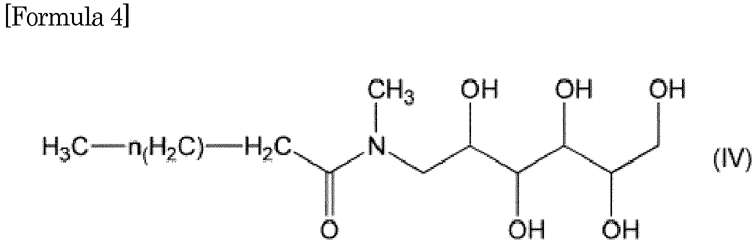 where n is 5 to 7. Specific examples of methylglucanamides include MEGA8 (octanoyl-N-methylglucamide), MEGA9 (nonanoyl-N-methylglucamide), and MEGA10 (decanoyl-N-methylglucamide).

The concentration of solubilizing agent contained in the first reagent may be suitably selected according to the kind of solubilizing agent used. For example, when a sarcosine derivative is used as a solubilizing agent, the concentration of solubilizing agent contained in the first reagent is 0.05 to 3.0 g/L and preferably 0.1 to 1.0 g/L. When a cholic acid derivative is used, the concentration of solubilizing agent contained in the first reagent is 0.1 to 10.0 g/L and preferably 0.2 to 2.0 g/L. When a methylglucanamide is used, the concentration of solubilizing agent contained in the first reagent is 1.0 to 8.0 g/L and preferably 2.0 to 6.0 g/L. When n-octyl ß-glucoside, sucrose monocaprate or N-formylmethylleucylalanine is used, the concentration of solubilizing agent contained in the first reagent is 0.01 to 50.0 g/L and preferably 0.05 to 30.0 g/L.

The pH of the first reagent is preferably 5.0 to 9.0, more preferably 6.5 to 7.5, and even more preferably 6.8 to 7.3. The pH of the first reagent can be adjusted with a buffer or a pH adjustor. Examples of buffers include Good's buffers such as HEPES, MOPS (3-morpholinopropanesulfonic acid) and MOPSO (2-hydroxy-3-morpholinopropanesulfonic acid), and phosphate buffers. Examples of pH adjustors include sodium hydroxide and hydrochloric acid.

The osmotic pressure of the first reagent can be suitably determined according to the kind of surfactant described above and the concentration thereof in the first reagent. A specific example of the osmotic pressure of the first reagent may be 10 to 600 mOsm/kg. The osmotic pressure of the first reagent may be adjusted by adding sugar, amino acid, sodium chloride, or the like to the first reagent. Specific examples of sugars include monosaccharides, polysaccharides, and sugar alcohols. Glucose and fructose are preferable as monosaccharides. Arabinose is preferable as a polysaccharide. Xylitol, sorbitol, mannitol, and ribitol are preferable as sugar alcohols. A sugar to be added to the first reagent is preferably a sugar alcohol and particularly preferably xylitol. When xylitol is added to the first reagent, the concentration of xylitol in the first reagent is preferably 1.0 to 75.0 g/L and particularly preferably 20.0 to 50.0 g/L. Specific examples of amino acids include valine, proline, glycine, and alanine, with glycine and alanine being particularly preferable. When glycine is added to the first reagent, the concentration of glycine in the first reagent is preferably 1.0 to 50.0 g/L and particularly preferably 10.0 to 30.0 g/L.

The electric conductivity of the first reagent is preferably 0.01 to 3 mS/cm and particularly preferably 0.1 to 2 mS/cm. In addition, a chelating agent, a preservative, or the like may be added to the first reagent. Examples of chelating agents include EDTA-2K and EDTA-3Na. Examples of preservatives include Proxel GXL (manufactured by Avecia) and Material TKM-A (API Corporation).

Due to the use of the first reagent, normal leukocytes and abnormal mononuclear leukocytes are more likely to be stained with a fluorescent dye that will be described below, and in addition, abnormal mononuclear leukocytes develop a difference in staining degree, size or other features of abnormal lymphocytes, atypical lymphocytes, and blasts. It is therefore possible based on the fluorescence signal (fluorescence intensity) and the scattered light signal (scattered light intensity) derived from hemocytes to distinguishably detect abnormal lymphocytes, atypical lymphocytes, and blasts in abnormal mononuclear leukocytes.

The second reagent is a stain solution for fluorescently staining nucleated cells in a blood sample. A fluorescent dye capable of staining nucleic acid is contained in the second reagent. There is no particular limitation on the fluorescent dye as long as it is capable of fluorescently staining nucleic acid. Such a dye barely stains erythrocytes that do not have nucleic acid, but stains nucleated hemocytes such as abnormal lymphocytes having nucleic acid. The fluorescent dye capable of staining nucleic acid can be suitably selected according to the light irradiated from a light source. Examples of fluorescent dyes capable of staining nucleic acid include propidium iodide, ethidium bromide, ethidium-acridine heterodimer, ethidium diazide, ethidium homodimer-1, ethidium homodimer-2, ethidium monoazide, trimethylenebis[[3-[[4-[[(3-methylbenzothiazol-3-ium)-2-yl]methylene]-1,4-dihydroquin oline]-l-yl]propyl]dimethylaminium] tetraiodide (TOTO-1), 4-[(3-methylbenzothiazol-2(3H)-ylidene)methyl]-1-[3-(trimethylaminio)propyl]quinolini um diiodide (TO-PRO-1), N,N,N',N'-tetramethyl-N,N'-bis[3-[4-[3-[(3-methylbenzothiazol-3-ium)-2-yl]-2-propenyli dene]-1,4-dihydroquinolin-1-yl]propyl]-1,3-propandiaminium tetraiodide (TOTO-3), 2- [3- [[1- [3-(trimethylaminio)propyl] -1,4-dihydroquinolin] -4-ylidene] -1-propenyl] -3-met hylbenzothiazol-3-ium diiodide (TO-PRO-3), and fluorescent dyes represented by structural formulas (V) to (XVIII) below.

### Structural formula (V)

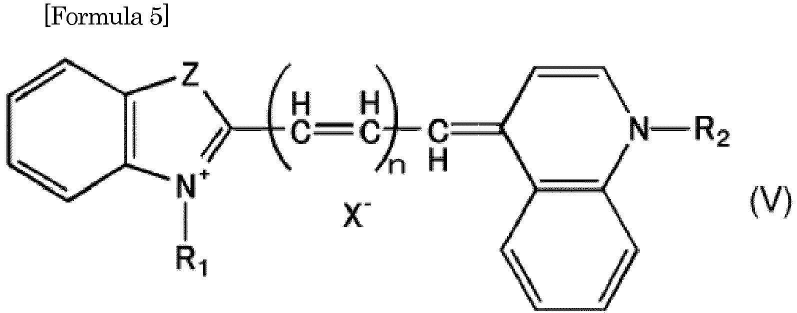

where R₁ and R₂ each are a lower alkyl group; n is 1 or 2; X⁻ is an anion; and Z is a sulfur atom, an oxygen atom, or a carbon atom substituted with a lower alkyl group.

In structural formula (V), the lower alkyl group is a linear or branched alkyl group having 1 to 6 carbon atoms. Specific examples of lower alkyl groups include a methyl group, an ethyl group, a propyl group, a butyl group, an iso-butyl group, a sec-butyl group, a tert-butyl group, a pentyl group, and a hexyl group, with a methyl group and an ethyl group being preferable. Z is preferably a sulfur atom. Examples of anions represented by X- include halogen ions (fluorine, chlorine, bromine, and iodine ions), boron halide ions (BF₄⁻, BCl₄⁻, BBr₄⁻, and the like), phosphorus compound ions, halogen oxoacid ions, fluorosulfuric acid ions, methylsulfuric acid ions, and ions of tetraphenylboron compounds having a haloaromatic ring or an alkyl group having a halogen as a substituent, with an iodine ion being preferable.

Among the fluorescent dyes represented by structural formula (V), a particularly preferable fluorescent dye capable of staining nucleic acid is NK-321 represented by structural formula (VI) below:

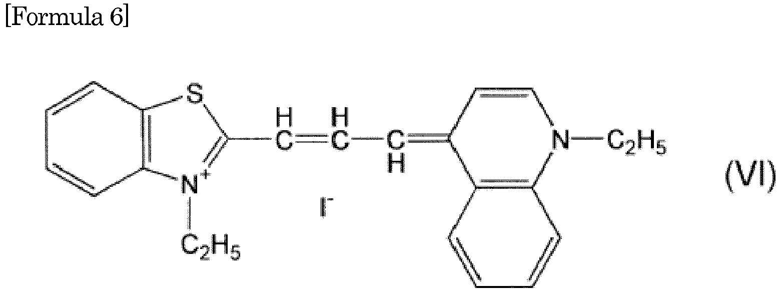

### Structural formula (VII)

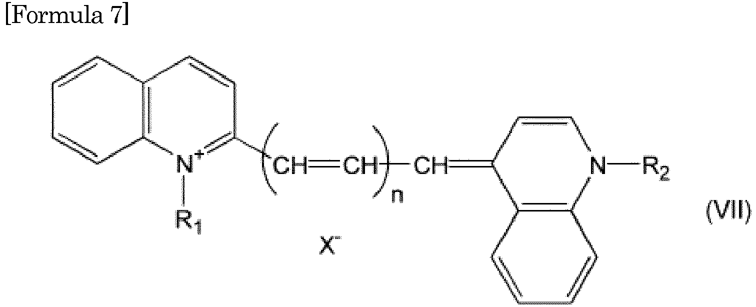

where R₁ and R₂ each are a lower alkyl group; n is 1 or 2; and X⁻ is an anion.

The lower alkyl group and the anion X⁻ in structural formula (VII) are the same as those in structural formula (V).

Among the fluorescent dyes represented by structural formula (VII), a particularly preferable fluorescent dye capable of staining nucleic acid is represented by structural formula (VIII) below:

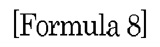

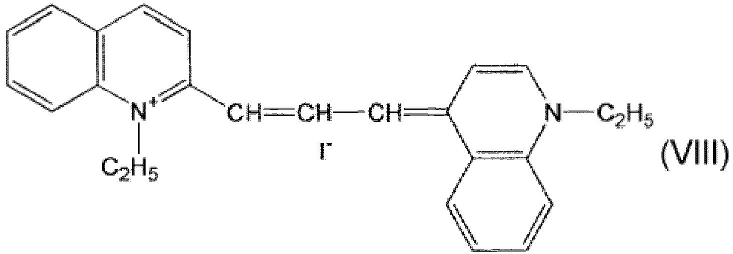

### Structural formula (IX)

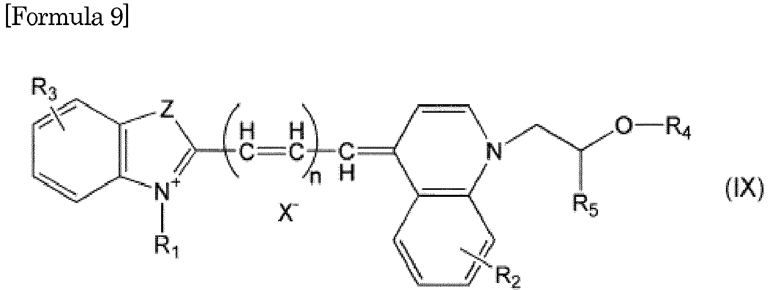

where R₁ is a hydrogen atom or a lower alkyl group; R₂ and R₃ each are a hydrogen atom, a lower alkyl group, or a lower alkoxy group; R₄ is a hydrogen atom, an acyl group, or a lower alkyl group; R₅ is a hydrogen atom or a lower alkyl group that may be substituted; Z is a sulfur atom, an oxygen atom, or a carbon atom substituted with a lower alkyl group; n is 1 or 2; and X⁻ is an anion.

The lower alkyl group and the anion represented by X⁻ in structural formula (IX) are the same as those in structural formula (V). The lower alkoxy group refers to an alkoxy group having 1 to 6 carbon atoms. Specific examples of lower alkoxy groups include a methoxy group, an ethoxy group, and a propoxy group, with a methoxy group and an ethoxy group being particularly preferable. The acyl group is preferably an acyl group derived from an aliphatic carboxylic acid. Specific examples of acyl groups include an acetyl group and a propionyl group, with an acetyl group being particularly preferable. Examples of substituents of the lower alkyl group that may be substituted include a hydroxyl group and halogen atoms (fluorine, chlorine, bromine, and iodine). The lower alkyl group that may be substituted may be substituted by 1 to 3 substituents. It is particularly preferable that the lower alkyl group that may be substituted is a lower alkyl group substituted with one hydroxyl group. Z is preferably a sulfur atom and X⁻ is preferably a bromine ion or BF₄⁻.

Among the fluorescent dyes represented by structural formula (IX), particularly preferable fluorescent dyes capable of staining nucleic acid are represented by any of the three structural formulas (X) to (XII) below:

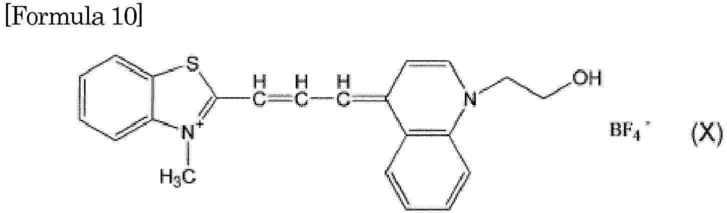

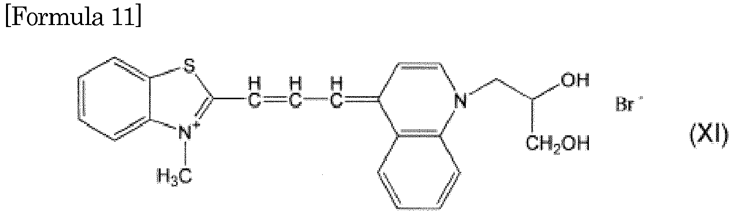

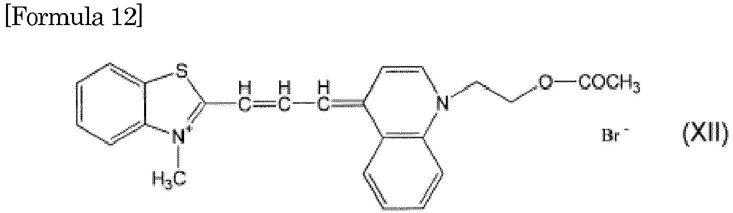

### Structural formula (XIII)

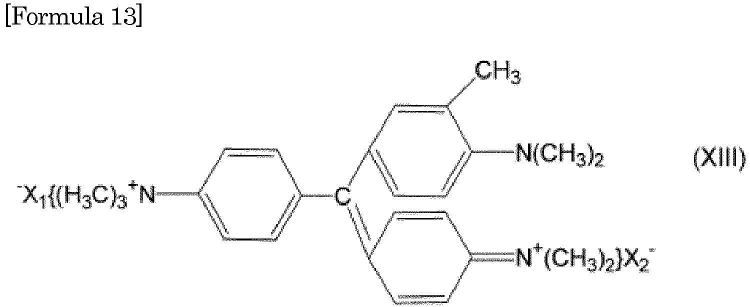

where X₁ and X₂ are independently Cl or I.

### Structural formula (XIV)

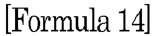

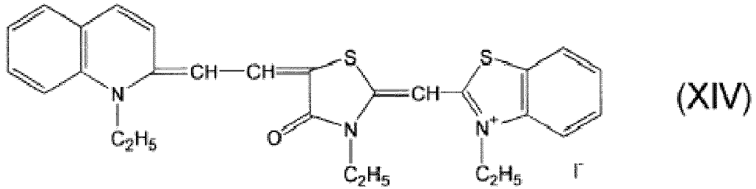

### Structural formula (XV) (NK-1570)

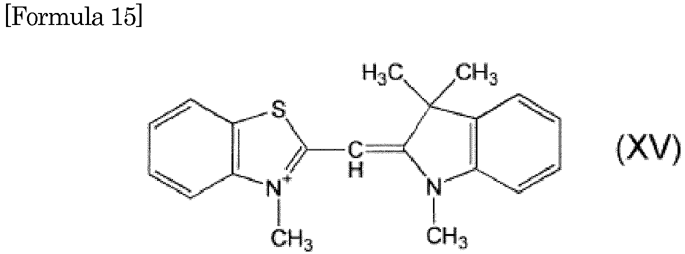

### Structural formula (XVI) (NK-1049)

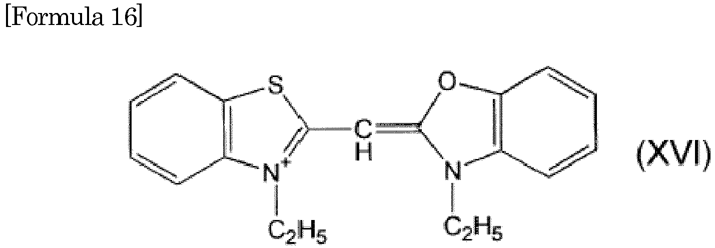

### Structural formula (XVII) (NK-98)

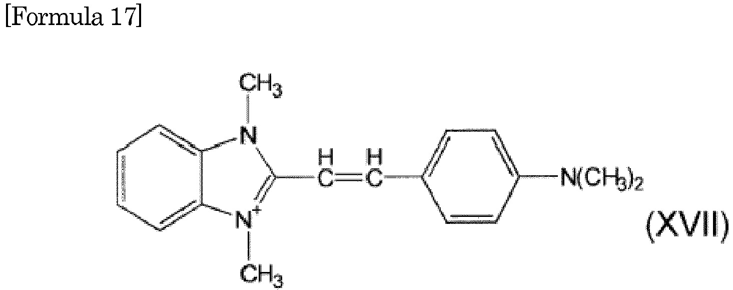

### Structural formula (XVIII) (NK-141)

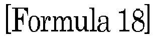

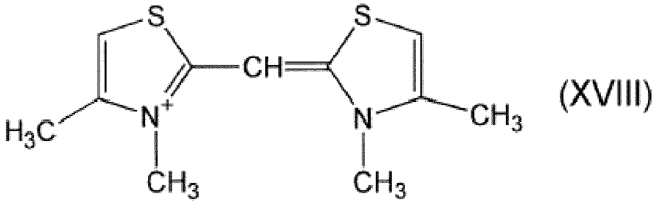

Among the above-described fluorescent dyes capable of staining nucleic acid, a particularly preferable fluorescent dye contained in the second reagent is NK-321 represented by the structural formula (XIX) below:

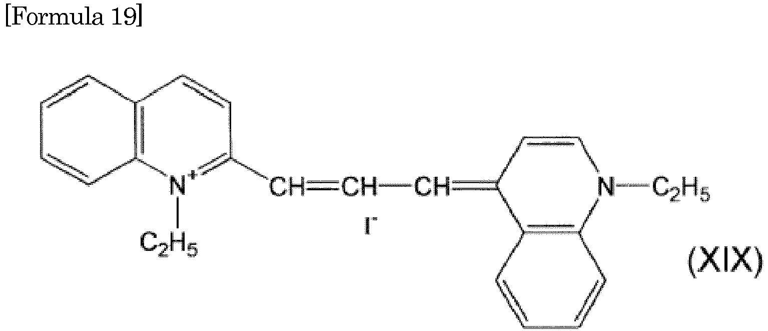

The concentration of above-described fluorescent dye capable of staining nucleic acid in the second reagent is preferably 10 to 500 mg/L and particularly preferably 30 to 100 mg/L. The second reagent may contain one or two or more fluorescent dyes capable of staining nucleic acid.

The detection portion 23 includes an optical detector D that can conduct a specimen measurement by flow cytometry. In the specimen measurement using the optical detector D, a measurement sample obtained by mixing the blood specimen, the first reagent, and the second reagent is supplied to the optical detector D, and optical information (fluorescence intensity, forward scattered light intensity, and side scattered light intensity) is detected in hemocytes in the measurement sample by the optical detector D at this time. The optical information obtained by the specimen measurement is supplied to the information processing unit 3, thereby determining whether any abnormal lymphocyte is present in the blood specimen and whether any blast is present therein.

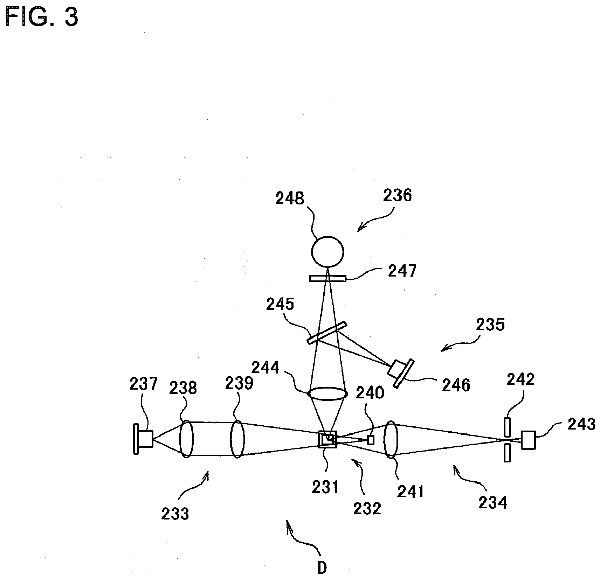

FIG. 3 shows a configuration outline of the optical detector D. The optical detector D feeds the measurement samples and the sheath fluid into a flow cell 231 to generate a liquid current in the flow cell 231, and measures the hemocytes contained in the liquid current flossing through the flow cell 231 by irradiating semiconductor laser light onto the hemocytes. The optical detector D includes a sheath flows system 232, a beam spot formation system 233, a forward scattered light receiving system 234, a side scattered light receiving system 235, and a fluorescence receiving system 236.

The sheath flows system 232 is configured to cause the measurement samples to flow through the flow cell 231 in such a state where the measurement samples are each enclosed in the sheath fluid. The beam spot formation system 233 is configured to allow light irradiated from a semiconductor laser 237 to be irradiated onto the flow cell 231 through a collimator lens 238 and a condenser lens 239. The beam spot formation system 233 also includes a beam stopper 240.

The forward scattered light receiving system 234 is configured to focus forward scattered light with a forward focusing lens 241, and receive the light that has passed through a pinhole 242 with a photodiode (forward scattered light receiving portion) 243.

The side scattered light receiving system 235 is configured to focus side scattered light with a side focusing lens 244, reflect a portion of the light at a dichroic mirror 245, and receive the reflected light with a photodiode (side scattered light receiving portion) 246.

Light scattering is a phenomenon that occurs when light changes the direction of its movement due to the presence of particles such as hemocytes in the movement direction as impediments. Information relating to the size and the material of the particles can be obtained by detecting such scattered light. In particular, information relating to the size of the particles (hemocytes) can be obtained from forward scattered light. Meanwhile, information about the interior of the particles can be obtained from side scattered light. When hemocyte particles are irradiated with laser light, the intensity of side scattered light is dependent on the complexity of the cell interior (the shape, size, and density of the nucleus, and the granular amount). Therefore, the intensity of these scattered light beams can be utilized for classification of leukocytes, detection of blasts, and the like.

The fluorescence receiving system 236 is configured to allow the light that has transmitted through the dichroic mirror 245 to further transmit through a spectral filter 247, and receive the transmitted light with an avalanche photodiode (fluorescence receiving portion) 248.

When a hemocyte that has been stained by a fluorescent substance is irradiated with light, the hemocyte emits light having a wavelength longer than the wavelength of the irradiated light. The intensity of fluorescence is increased if the hemocyte has been stained well, and information relating to the staining degree of the hemocyte can be obtained by measuring the fluorescence intensity Accordingly, the difference in fluorescence intensity can be utilized for detection of leukocytes, detection of abnormal lymphocytes, detection of blasts, and the like.

Referring back to FIG. 2, the configuration of the specimen container carrying portion 25 will be described next. The specimen container carrying portion 25 includes a hand portion 25a that can grip the specimen container T. The hand portion 25a includes a pair of gripping members arranged facing each other, and can move these gripping members toward and away from each other. The specimen container T can be gripped by the gripping members by moving the gripping members toward each other with the specimen container T interposed therebetween. Furthermore, the specimen container carrying portion 25 can move the hand portion 25a in the up-down direction and the front-back direction (Y direction), and also can oscillate the hand portion 25a. This allows the specimen container T housed in the sample rack L and located at the specimen supply position 43a to be gripped by the hand portion 25a. In this state, the hand portion 25a is moved upward to pull out the specimen container T from the sample rack L. Then, the specimen in the specimen container T can be agitated by oscillating the hand portion 25a.

The specimen container carrying portion 25 also includes a specimen container setting portion 25b having a hole into which the specimen container T can be inserted. After completion of agitation, the specimen container T gripped by the hand portion 25a described above is moved such that the gripped specimen container T is inserted into the hole of the specimen container setting portion 25b. Then, the gripping members are moved away from each other, thereby releasing the specimen container T from the hand portion 25a and setting the specimen container T in the specimen container setting portion 25b. The specimen container setting portion 25b can be moved horizontally in Y1 and Y2 directions in FIG. 2 by the power of a stepping motor (not shown).

A bar code reading portion 26 is provided inside the measurement unit 2. The specimen container setting portion 25b can be moved to a bar code reading position 26a in the vicinity of the bar code reading portion 26 and to the suction position 21a where the specimen is suctioned by the specimen suction portion 21. When the specimen container setting portion 25b is moved to the bar code reading position 26a, the bar code of the specimen is read by the bar code reading portion 26. When the specimen container setting portion 25b is moved to the suction position, the specimen is suctioned by the specimen suction portion 21 from the specimen container T that has been set.

### Configuration of Information Processing Unit

Next, the configuration of the information processing unit 5 will be described. The information processing unit 5 is configured by a computer. FIG. 4 is a block diagram showing the configuration of the information processing unit 5. The information processing unit 5 can be implemented by a computer 5a. As shown in FIG. 4, the computer 5a includes a body 51, an image display portion 52, and an input portion 53. The body 51 includes a CPU 51a, a ROM 51b, a RAM 51c, a hard disk 51d, a readout device 51e, an input/output interface 51f, a communication interface 51g, and an image output interface 51h. The CPU 51a, the ROM 51b, the RAM 51c, the hard disk 51d, the readout device 51e, the input/output interface 51f, the communication interface 51g, and the image output interface 51h are connected by a bus 51j.

The CPU 51a can execute a computer program loaded into the RAM 51c. The computer 5a functions as the information processing unit 5 by the CPU 51a executing a computer program 54a for blood analysis and control of the measurement unit 2 and the specimen carrying unit 4 as will be described later.

Various computer programs, including, for example, an operating system and application programs, for being executed by the CPU 51a, and the data used for execution of such computer programs are installed in the hard disk 51d. The computer program 54a for enabling the CPU to execute processing described later is also installed in the hard disk 51d. The computer program 54a is an event-driven computer program.

The readout device 51e is configured by a flexible disk drive, a CD-ROM drive, a DVD-ROM drive, or the like, and can read out the computer programs or data recorded in a portable recording medium 54. The computer program 54a for enabling the computer to function as the information processing unit 5 is stored in the portable recording medium 54. The computer 5a can read out the computer program 54a from the portable recording medium 54, and install the computer program 54a in the hard disk 51d.

For example, a multitasking operating system such as Windows (registered trademark) manufactured and sold by Microsoft Corporation, US is installed in the hard disk 51d. The following description is given assuming that the computer program 54a according to this embodiment runs on that operating system.

The input/output interface 51f is configured, for example, by a serial interface such as USB, IEEE1394, or RS-232C, a parallel interface such as SCSI, IDE, or IEEE1284, and an analog interface made up of a D/A converter, an A/D converter, and the like. An input portion 53 made up of a keyboard and a mouse is connected to the input/output interface 51f, and the user can input data into the computer 5a using the input portion 53. Furthermore, the input/output interface 51f is connected to the measurement unit 2 and the specimen carrying unit 4. This enables the information processing unit 5 to control each of the measurement unit 2 and the specimen carrying unit 4.

The communication interface 51g is an Ethernet (registered trademark) interface. The communication interface 51g is connected to a host computer 6 via a LAN (see FIG. 2). The computer 5a can transmit and receive data via the communication interface 51g to and from the host computer 6 connected to the LAN using a predetermined communications protocol.

### Specimen Measuring Operation of Blood Analyzer

Hereinafter, the operation of the blood analyzer 1 according to this embodiment will be described. The blood analyzer 1 can perform a specimen measurement using the optical detector D. The specimen measurement steps include a measurement step in which a measurement sample is measured and a data processing step in which the measurement data obtained in the measurement step is subjected to analysis processing.

First, the sample rack L holding the specimen container T is placed on the specimen carrying unit 4 by the operator. The sample rack L is carried by the specimen carrying unit 4, and the specimen container T housing a specimen to be measured is positioned in the specimen supply position 43a. Next, the specimen container T is gripped by the hand portion 25a of the measurement unit 2, and the specimen container T is taken out from the sample rack L. The hand portion 25a then causes oscillating movement, and thereby the specimen inside the specimen container T is agitated. Next, the specimen container T is inserted into the specimen container setting portion 25b, and the specimen container setting portion 25b is moved in the Y direction. After the bar code of the specimen is read by the bar code reading portion 26, the specimen container T reaches the suction position. Then, a measurement step described below is performed.

### Measurement Step

The measurement step will be described first. In the measurement step, the blood analyzer 1 mixes a whole blood specimen (17.0 µL), a first reagent (1000 µL), and a second reagent (20 µL) to prepare a measurement sample, and measures the measurement sample by flow cytometry using the optical detector D.

In this embodiment, the following reagents are used as the first reagent.

### First Reagent

| | |
|---|---|
| MOPS | 2.09 g/L |
| polyoxyethylene (20) oleyl ether | 1.25 g/L |
| sodium N-lauroylsarcosinate | 0.268 g/L |
| EDTA-2K | 0.5 g/L |

The above-listed ingredients were mixed, and NaOH was further added to adjust the pH to 7.3. The osmotic pressure of the first reagent was 37 mOsm/Kg, and the electric conductivity thereof was 0.745 mS/cm.

### Second Reagent

| | |
|---|---|
| NK-321 | 50 mg/L |

NK-321 (50 mg/L) dissolved in ethylene glycol was used as the second reagent.

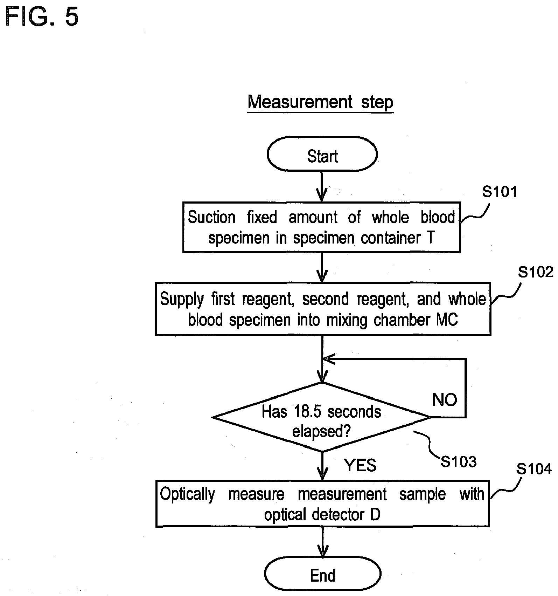

FIG. 5 is a flowchart illustrating the procedure of operation performed by the blood analyzer 1 in the measurement step. First, the CPU 51a controls the specimen suction portion 21 to suction a fixed amount of the whole blood specimen in the specimen container T with the suction tube 211 (step S101). Specifically, in the processing of step S101, the suction tube 211 is inserted into the specimen container T, and a fixed amount (39.0 µL) of the whole blood specimen is suctioned by driving the syringe pump.

Next, the CPU 51a controls the measurement unit 2 to supply, to the mixing chamber MC, the first reagent (1000 µL) from the reagent container 221a, the second reagent (20 µL) from the reagent container 221b, and the whole blood specimen (17.0 µL) from the suction tube 211 (step S102). In this step S102, the specimen supplied to the mixing chamber MC is a portion of the whole blood specimen suctioned by the suction tube 211 in the step S101 described above.

Next, the CPU 51a waits 18.5 seconds and determines whether 18.5 seconds have elapsed since the supply of the first reagent, the second reagent and the whole blood specimen to the mixing chamber MC (step S103). Here, the mixing chamber MC has been heated to 34.0°C by the heater. Thus, the mixed solution of the first reagent, the second reagent and the blood specimen is heated at 34.0°C for 18.5 seconds to prepare the measurement sample.

Then, optical measurement is conducted on the measurement sample with the optical detector D (step S104). Specifically, in the processing of step S104, the measurement sample and the sheath fluid are simultaneously supplied to the flow cell 231 of the optical detector D. At that time, forward scattered light is received by the photodiode 243, side scattered light is received by the photodiode 246, and fluorescence is received by the avalanche photodiode 248. In this optical measurement, a red semiconductor laser having an excitation wavelength of 633 nm is used as a light source to detect fluorescence (red fluorescence) having a wavelength of 650 nm or more as a fluorescence signal. Output signals (analog signals) output from these various light-receiving elements of the optical detector D are converted into digital signals by an A/D converter (not shown), and then converted into measurement data that is digital data through predetermined signal processing. The measurement data is transmitted to the information processing unit 5. In this signal processing, a forward scattered light signal (forward scattered light intensity), a side scattered light signal (side scattered light intensity), and a fluorescence signal (fluorescence intensity) are obtained as feature parameters contained in the measurement data. This completes the measurement step. As will be described later, the CPU 51a of the information processing unit 5 performs predetermined analysis processing on the measurement data to detect abnormal lymphocytes or blasts and generate analysis result data containing this detection result, and stores the analysis result data in the hard disk 51d.

### Data Processing Step

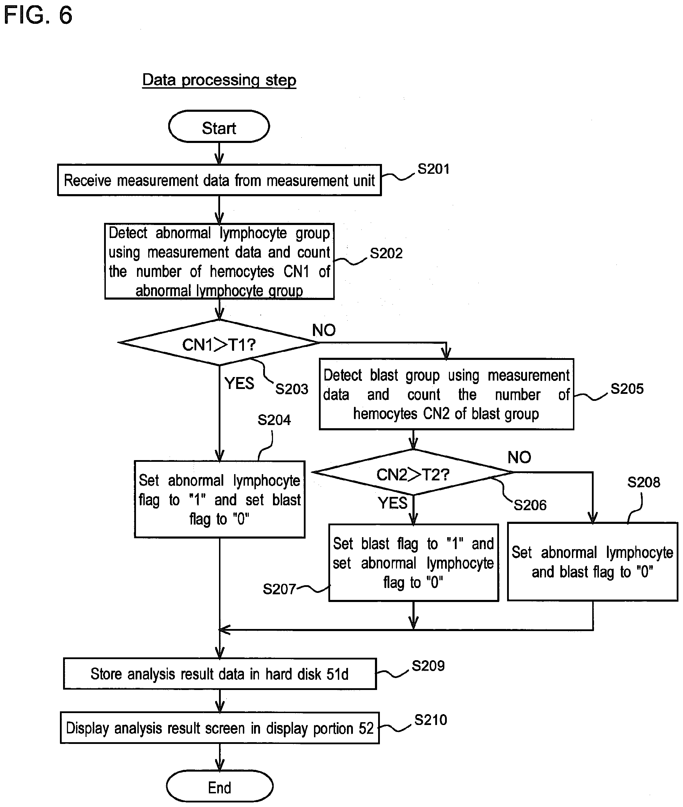

Next, the data processing step will be described. FIG. 6 is a flowchart showing the procedure of processing in the data processing step performed by the blood analyzer according to this embodiment. The information processing unit 5 of the blood analyzer 1 receives the measurement data from the measurement unit 2 (step S201). The computer program 54a, which is executed by the CPU 51a, is an event-driven program, and the processing of step S202 is invoked upon occurrence of an event of receiving the measurement data.

In the step S202, the CPU 51a detects a cell group of abnormal lymphocytes (hereinafter, referred to as an "abnormal lymphocyte group") using the measurement data, and counts the number of hemocytes CN1 contained in the detected abnormal lymphocyte group (step S202).

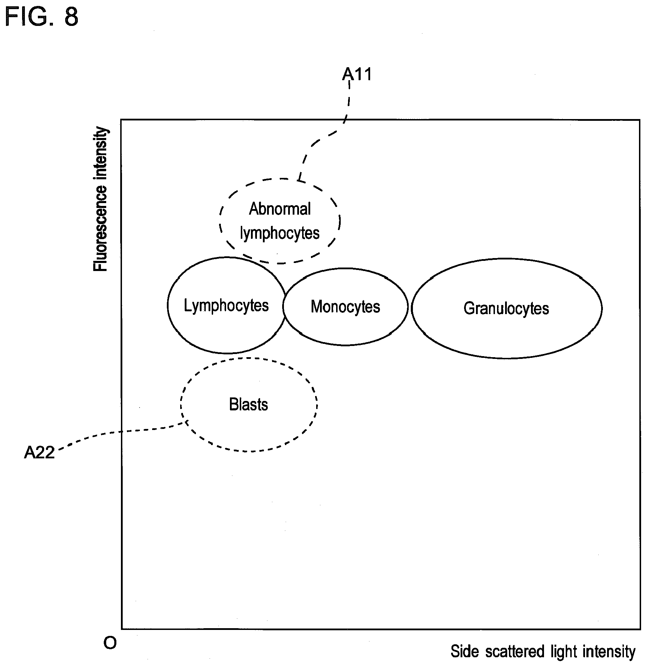

Processing of step S202 will be described with reference to a schematic diagram. FIG. 7 is a schematic diagram showing a scattergram obtained when measurement data of forward scattered light intensity and fluorescence intensity received from the measurement unit 2 is plotted on a two-dimensional plane, and FIG. 8 is a schematic diagram showing a scattergram obtained when measurement data of fluorescence intensity and side scattered light intensity received from the measurement unit 2 is plotted on a two-dimensional plane. These scattergrams show that intensity becomes larger with a distance from the origin 0 shown in the drawings. A cluster of blasts, a cluster of granulocytes (hemocyte group includes neutrophils, eosinophils and basophils), a cluster of lymphocytes, and a cluster of monocytes appear in the scattergram shown in FIG. 7. Also, a cluster of blasts, a cluster of granulocytes, a cluster of lymphocytes, and a cluster of monocytes appear in the scattergram shown in FIG. 8.

In this embodiment, the range of forward scattered light intensity and fluorescence intensity indicated by the dashed line in FIG. 7 is defined as a detection area A1 for an abnormal lymphocyte group. As shown in FIG. 7, the detection area A1 is set in a portion with a fluorescence intensity higher than that in the area where lymphocytes and monocytes appear. As a result of experiments using clinical specimens and detailed investigation of the results of the experiments, the present inventors have found that abnormal lymphocytes appear in the detection area A1 and the area A11 shown in FIG. 8, and that blasts appear in the area A21 shown in FIG. 7 and the area A22 shown in FIG. 8. The area A11 shown in FIG. 8 is also an area with a fluorescence intensity higher than that in the area where lymphocytes and monocytes appear. The area A21 shown in FIG. 7 and the area A22 shown in FIG. 8 are areas with a fluorescence intensity lower than that in the area where lymphocytes and monocytes appear. Furthermore, as a result of detailed evaluation of the results of the experiments, the inventors found that atypical lymphocytes appear in none of the areas A1, A11, A21 and A22 described above, but appear in the area where normal leukocytes appear. Therefore, it has been found that the area A1 shown in FIG. 7 and the area A11 shown in FIG. 8 can be used to distinguishably detect abnormal lymphocytes from atypical lymphocytes and blasts. In step S202, any cell group that appears within the above-described detection area A1 is detected as an abnormal lymphocyte group, and the number of hemocytes CN1 is counted.

Next, the CPU 51a determines whether CN1 is greater than a predetermined threshold T1 (step S203). The threshold T1 is a reference value for determining whether any abnormal lymphocyte is present in a blood specimen. In step S203, if CN1 is greater than the threshold T1, it is determined that abnormal lymphocytes are present in the blood specimen. If CN1 is less than or equal to the threshold T1, it is determined that no abnormal lymphocytes are present in the blood specimen.

If CN1 > T1 in step S203 (YES in step S203), the CPU 51a sets an abnormal lymphocyte flag provided in the RAM 51c to "1" and sets a blast flag to "0" (step S204). Here, the abnormal lymphocyte flag is a flag indicating the presence or absence of abnormal lymphocytes in a blood specimen. The abnormal lymphocyte flag indicates the presence of abnormal lymphocytes if it is set to "1", and indicates the absence of abnormal lymphocytes if it is set to "0". The blast flag is a flag indicating the presence or absence of blasts in the blood specimen. The blast flag indicates the presence of blasts if it is set to "1", and indicates the absence of blasts if it is set to "0". Then, the processing executed by the CPU 51a moves to step S209.

On the other hand, if CN1 ≤ T1 in step S203 (NO in step S203), the CPU 51a detects a cell group of blasts (hereinafter, referred to as a "blast group,") using the measurement data, and counts the number of hemocytes CN2 contained in the detected blast group (step S205).

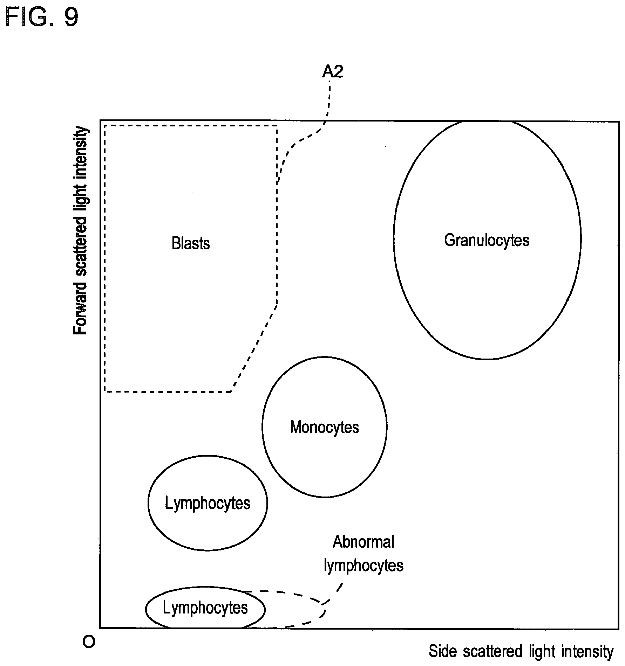

The processing of step S205 will be described in detail. FIG. 9 is a schematic diagram showing a scattergram obtained when measurement data of forward scattered light intensity and side scattered light intensity received from the measurement unit 2 is plotted on a two-dimensional plane. A cluster of granulocytes, a cluster of lymphocytes, and a cluster of monocytes appear in the scattergram shown in FIG. 9. In this embodiment, the range of forward scattered light intensity and side scattered light intensity indicated by the dashed line in FIG. 9 is defined as a detection area A2 for a blast group. As shown in FIG. 9, the detection area A2 is set in a portion with a forward scattered light intensity higher than that in the area where lymphocytes appear. As a result of experiments using clinical specimens and detailed investigation of the results of the experiments, the present inventors have found that blasts appear in the detection area A2, and that abnormal lymphocytes and atypical lymphocytes do not appear in the detection area A2. Therefore, use of the detection area A2 allows blasts to be detected distinguishably from abnormal lymphocytes and atypical lymphocytes. In step S205, any cell group that appears within the above-described detection area A2 is detected as a blast group, and the number of hemocytes CN2 is counted. Note that, as described above, the present inventors also have found that blasts appear in the area A21 shown in FIG. 7 and the area A22 shown in FIG. 8, and abnormal lymphocytes and atypical lymphocytes do not appear in these areas. Therefore, the area A21 or the area A22 may be used to distinguishably detect blasts from abnormal lymphocytes and atypical lymphocytes.

Next, the CPU 51a determines whether CN2 is greater than a predetermined threshold T2 (step S206). The threshold T2 is a reference value for determining whether any blast is present in a blood specimen. In step S206, if CN2 is greater than the threshold T2, it is determined that blasts are present in the blood specimen. If CN2 is less than or equal to the threshold T2, it is determined that no blasts are present in the blood specimen.

If CN2 > T2 (YES in step S206), the CPU 51a sets the blast flag provided in the RAM 51c to "1" and sets the abnormal lymphocyte flag to "0" (step S207). Then, the processing executed by the CPU 51a moves to step S208.

On the other hand, if CN2 ≤ T2 (NO in step S206), the CPU 51a sets each of the abnormal lymphocyte flag and the blast flag provided in the RAM 51c to "0" (step S208). Then, the processing executed by the CPU 51a moves to step S209.

In the step S209, the CPU 51a stores the thus obtained analysis result (including the abnormal lymphocyte flag and blast flag) in the hard disk 51d. Next, the CPU 51a causes the image display portion 52 to display an analysis result screen showing the analysis result stored in the hard disk 51d (step S210), and ends the data processing.

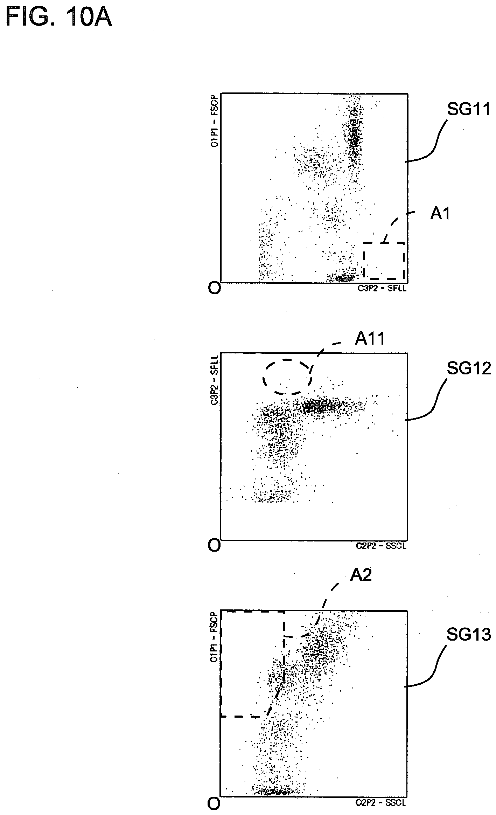

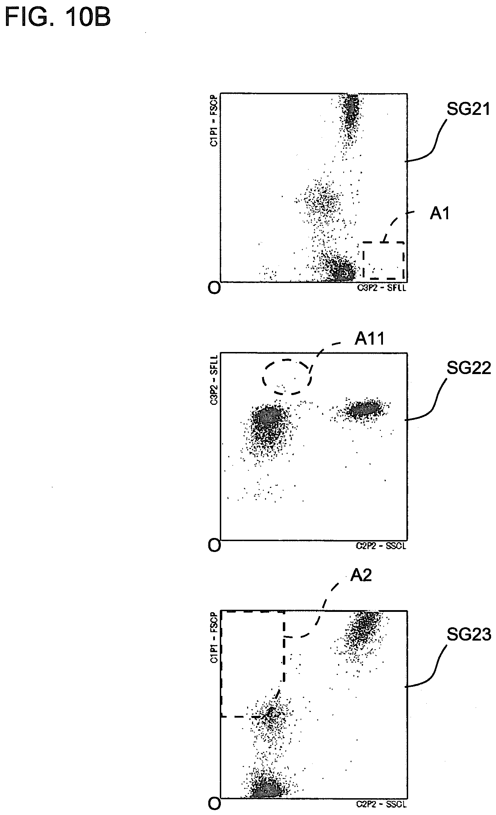

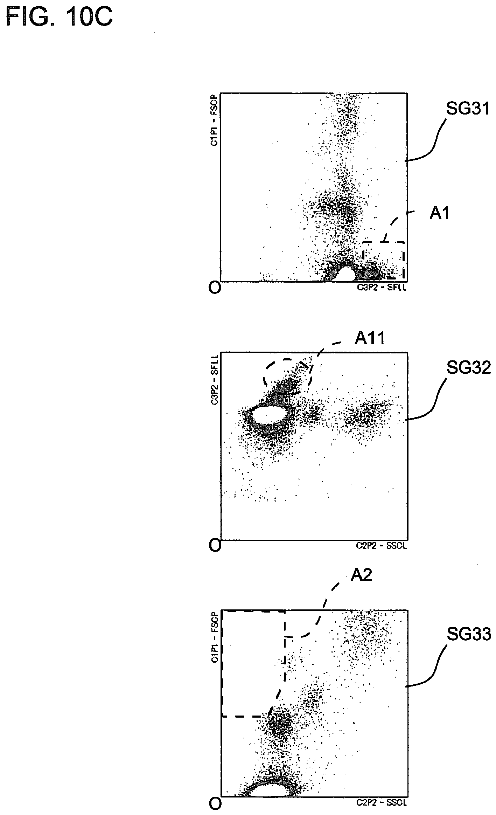

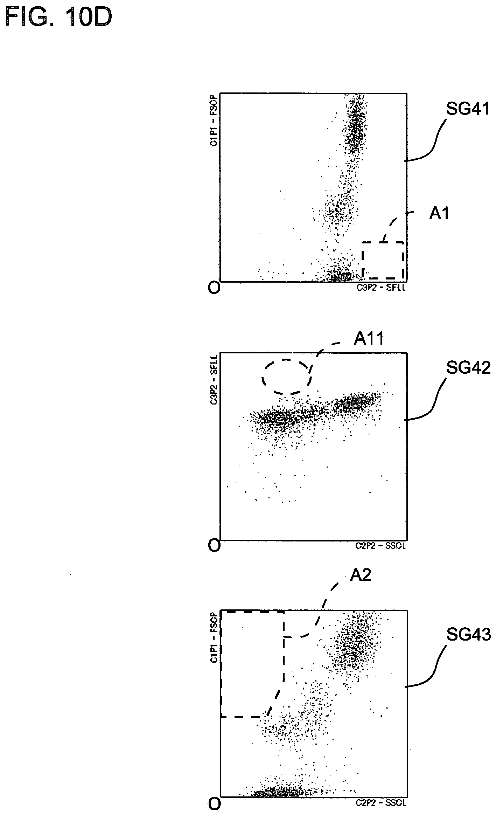

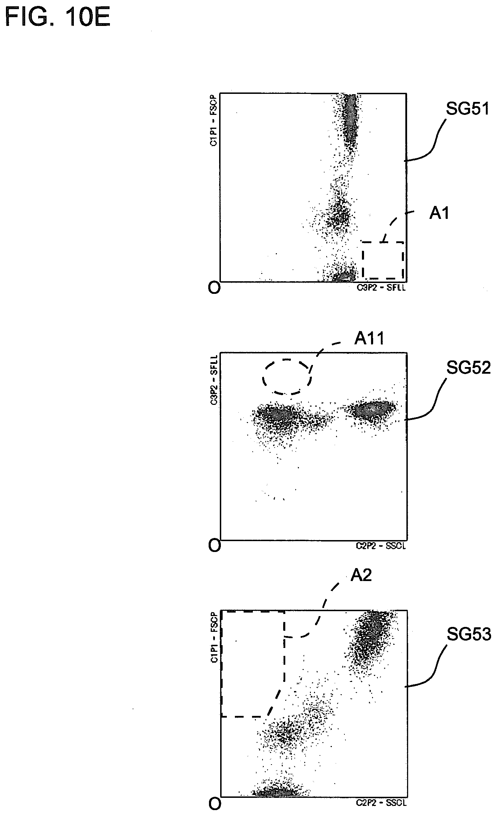

Next, examples of scattergrams obtained when measuring a specific blood specimen are shown, and the analysis of the measurement data performed by the blood analyzer 1 according to this embodiment will be described. FIGS. 10A to 10E show examples of scattergrams obtained in substantially the same measurement condition as the measurement condition by the blood analyzer 1. FIG. 10A shows examples of scattergrams obtained when measuring a blood specimen A containing myeloblasts. FIG. 10B shows examples of scattergrams obtained when measuring a blood specimen B containing lymphoblasts FIG. 10C shows examples of scattergrams obtained when measuring a blood specimen C containing abnormal lymphocytes. FIG. 10D shows examples of scattergrams obtained when measuring a blood specimen D containing atypical lymphocytes. FIG. 10E shows examples of scattergrams obtained when measuring a normal blood specimen E.

As shown in FIG. 10A, particles corresponding to the detected hemocytes are substantially absent (they are present in a very small amount, but the number of hemocytes does not exceed the threshold T1) in the detection area A1 for the abnormal lymphocytes in a scattergram SG11 of forward scattered light intensity and fluorescence intensity (scattergram for abnormal lymphocyte detection) in the measurement data of the blood specimen A. FIG. 10A also shows a scattergram SG12 of fluorescence intensity and side scattered light intensity in the measurement data of the blood specimen A for reference. It can be seen that in the scattergram SG12 as well, hemocytes are substantially absent in the area A11 where abnormal lymphocytes appear. Furthermore, particles corresponding to the detected hemocytes are present in the detection area A2 for blasts in a scattergram SG13 of forward scattered light intensity and side scattered light intensity (scattergram for blast detection) in the measurement data of the blood specimen A. That is, as can be seen from the scattergram SG13, the blast group is detected in the blood specimen A. Thus, CN1 ≤ T1 and CN2 > T2 when the blood analyzer 1 according to this embodiment analyses the blood specimen A, and therefore it is determined that blasts are present.

When the blood specimen A was checked by hand method (visual check using a microscope), the ratio of the number of myeloblasts to the number of total leukocytes was 15%, and the ratio of the number of each of lymphoblasts, abnormal lymphocytes and atypical lymphocytes to the number of total leukocytes was 0%. Therefore, it can be seen that detection of blasts in the blood specimen A by the blood analyzer 1 is appropriate.

As shown in FIG. 10B, particles corresponding to the detected hemocytes are substantially absent in the detection area A1 for the abnormal lymphocytes in a scattergram SG21 for abnormal lymphocyte detection in the measurement data of the blood specimen B. FIG. 10B also shows a scattergram SG22 of fluorescence intensity and side scattered light intensity in the measurement data of the blood specimen B for reference. It can be seen that in the scattergram SG22 as well, hemocytes are substantially absent in the area A11 where abnormal lymphocytes appear. Furthermore, particles corresponding to the detected hemocytes are present in the detection area A2 for blasts in a scattergram SG23 for blast detection in the measurement data of the blood specimen B. That is, as can be seen from the scattergram SG23, the blast group is detected in the blood specimen B. Thus, CN1 ≤ T1 and CN2 > T2 when the blood analyzer 1 according to this embodiment analyses the blood specimen B, and therefore it is determined that blasts are present.

When the blood specimen B was checked by hand method, the ratio of the number of lymphoblasts to the number of total leukocytes was 23.5%, and the ratio of the number of each of myeloblasts, abnormal lymphocytes and atypical lymphocytes to the number of total leukocytes was 0%. Therefore, it can be seen that detection of blasts in the blood specimen B by the blood analyzer 1 is appropriate.

As shown in FIG. 10C, particles corresponding to the detected hemocytes are present in the detection area A1 for the abnormal lymphocytes in a scattergram SG31 for abnormal lymphocyte detection in the measurement data of the blood specimen C. FIG. 10C also shows a scattergram SG32 of fluorescence intensity and side scattered light intensity in the measurement data of the blood specimen C for reference. It can be seen that in the scattergram SG32 as well, hemocytes are present in the area A11 where abnormal lymphocytes appear. Furthermore, particles corresponding to the detected hemocytes are substantially absent in the detection area A2 for blasts in a scattergram SG33 for blast detection in the measurement data of the blood specimen C. That is, as can be seen from the scattergrams SG31 and SG32, the abnormal lymphocyte group is detected in the blood specimen C. Thus, CN1 > T1, when the blood analyzer 1 according to this embodiment analyses the blood specimen C, and therefore it is determined that abnormal lymphocytes are present.

When the blood specimen C was checked by hand method, the ratio of the number of abnormal lymphocytes to the number of total leukocytes was 9%, and the ratio of the number of each of myeloblasts, lymphoblasts and atypical lymphocytes to the number of total leukocytes was 0%. Therefore, it can be seen that detection of abnormal lymphocytes in the blood specimen C by the blood analyzer 1 is appropriate.

As shown in FIG. 10D, particles corresponding to the detected hemocytes are substantially absent in the detection area A1 for the abnormal lymphocytes in a scattergram SG41 for abnormal lymphocyte detection in the measurement data of the blood specimen D. FIG. 10D also shows a scattergram SG42 of fluorescence intensity and side scattered light intensity in the measurement data of the blood specimen D for reference. It can be seen that in the scattergram SG42 as well, hemocytes are substantially absent in the area A11 where abnormal lymphocytes appear. Furthermore, particles corresponding to the detected hemocytes are substantially absent in the detection area A2 for blasts in a scattergram SG43 for blast detection in the measurement data of the blood specimen D. That is, as can be seen from the scattergram SG41 and SG43, neither the abnormal lymphocyte group nor the blast group is detected in the blood specimen D. Thus, CN1 ≤ T1 and CN2 ≤ T2 when the blood analyzer 1 according to this embodiment analyses the blood specimen D, and therefore it is determined that abnormal lymphocytes and blasts are absent.

When the blood specimen D was checked by hand method, the ratio of the number of atypical lymphocytes to the number of total leukocytes was 7%, and the ratio of the number of each of myeloblasts, lymphoblasts and abnormal lymphocytes to the number of total leukocytes was 0%. Therefore, it can be seen that no detection of both blasts and abnormal lymphocytes in the blood specimen D by the blood analyzer 1 is appropriate.

As described above, when a blood specimen containing abnormal lymphocytes, but not containing blasts and atypical lymphocytes is measured by the blood analyzer 1, abnormal lymphocytes are detected and blasts are not detected. Similarly, when a blood specimen containing blasts, but not containing abnormal lymphocytes and atypical lymphocytes is measured by the blood analyzer 1, blasts are detected and abnormal lymphocytes are not detected. Furthermore, when a blood specimen containing atypical lymphocytes, but not containing abnormal lymphocytes and blasts is measured by the blood analyzer 1, neither abnormal lymphocytes nor blasts are detected. That is, with the blood analyzer 1 according to the embodiment, it is possible to detect abnormal lymphocytes and blasts separately.

Abnormal lymphocytes, blasts, and atypical lymphocytes do not appear in normal peripheral blood. As shown in FIG. 10E, particles corresponding to the detected hemocytes are substantially absent in the detection area A1 for the abnormal lymphocytes in a scattergram SG51 for abnormal lymphocyte detection in the measurement data of the blood specimen E. FIG. 10E also shows a scattergram SG52 of fluorescence intensity and side scattered light intensity in the measurement data of the blood specimen E for reference. It can be seen that in the scattergram SG52 as well, hemocytes are substantially absent in the area A11 where abnormal lymphocytes appear. Furthermore, particles corresponding to the detected hemocytes are substantially absent in the detection area A2 for blasts in a scattergram SG53 for blast detection in the measurement data of the blood specimen E. As can be seen from the scattergrams SG51 and SG53, neither the abnormal lymphocyte group nor the blast group is detected in the blood specimen E. Thus, CN1 ≤ T1 and CN2 ≤ T2 when the blood analyzer 1 according to this embodiment analyses the blood specimen E, and therefore it is determined that the abnormal lymphocytes and the blasts are absent.

When the blood specimen E was checked by hand method, the ratio of the number of each of myeloblasts, lymphoblasts, abnormal lymphocytes, and atypical lymphocytes to the number of total leukocytes was 0%. Therefore, it can be seen that detection of neither blasts nor abnormal lymphocytes in the blood specimen E by the blood analyzer 1 is appropriate.

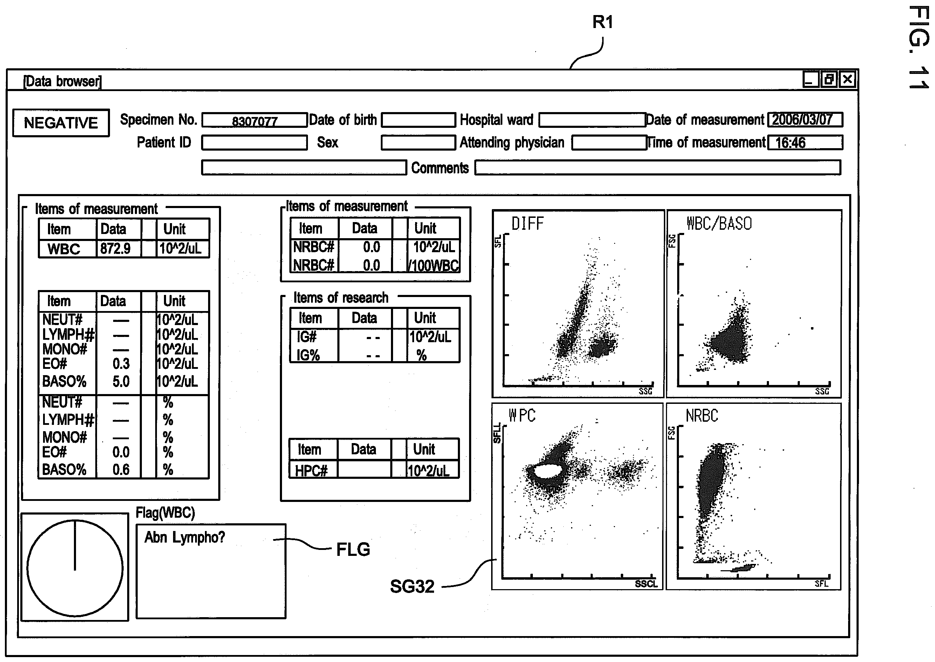

FIG. 11 is a diagram showing an example of an analysis result screen of the blood analyzer 1. FIG. 11 shows an analysis result screen output when the blood analyzer 1 analyses the blood specimen C. As described above, the blood specimen C contains abnormal lymphocytes, but does not contains blasts and atypical lymphocytes. As shown in FIG. 11, the measured numeric data of the measurement items (WBC, RBC, PLT, etc.) are displayed on an analysis result screen R1. In the analysis result data relating to the blood specimen C, the abnormal lymphocyte flag is set to "1", and the blast flag is set to "0". Accordingly, as shown in FIG. 11, the indication "Abn Lympho?", which is information indicating the possibility that abnormal lymphocytes are present, is added to the Flag field FLG on the analysis result screen R₁ for the blood specimen C. Although not shown here, when the blast flag is set to "1" and the abnormal lymphocyte flag is set to "0", "Blasts?" is indicated in the flag indication field FLG. Furthermore, when both the abnormal lymphocyte flag and the blast flag are set to "0", neither "Abn Lympho?" nor "Blasts?" above will be indicated on the analysis result screen. This enables the operator to understand whether any abnormal lymphocyte is detected in the blood specimen, and whether any blast is detected in the blood specimen, by just looking at the analysis result screen. In addition, the scattergram SG32 of fluorescence intensity and side scattered light intensity of the measurement data is indicated on the analysis result screen R1. By referring to the scattergram, the operator can understand the basis of the detection results obtained by the blood analyzer 1 for abnormal lymphocytes and blasts. The operator can also determine the validity of the detection results for abnormal lymphocytes and blasts obtained by the blood analyzer 1.

With the configuration described above, the blood analyzer 1 can detect abnormal lymphocytes and blasts separately by measuring, with the optical detector D, the measurement sample prepared by mixing the blood specimen, the first reagent containing a hemolyzing agent, and the second reagent containing a fluorescent dye for staining nucleic acid.

### Embodiment 2

Hereinafter, a blood analyzer according to this embodiment will be described.

### Configuration of Blood Analyzer

First, configuration of a blood analyzer according to this embodiment will be described.

### Configuration of Measurement Unit

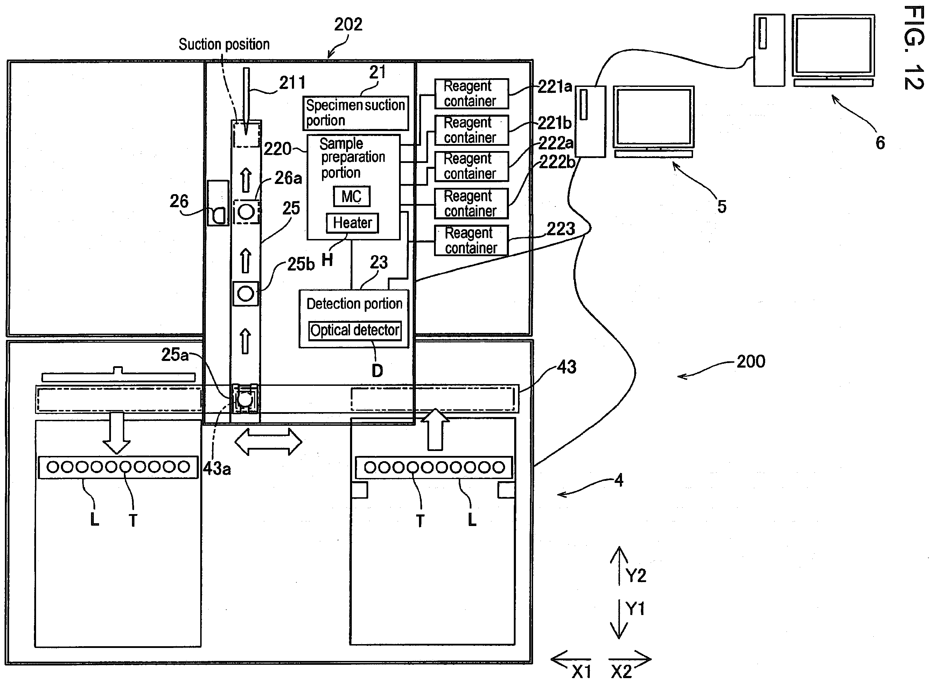

The configuration of the measurement unit will be described. FIG. 12 is a block diagram showing the configuration of the measurement unit according to this embodiment. As shown in FIG. 12, a blood analyzer 200 according to this embodiment includes the measurement unit 202. The measurement unit 202 is provided with a sample preparation portion 220, and two mixing chamber MC1 and MC2 is provided in the sample preparation portion 220. The suction tube 211 suctions a predetermined amount of a whole blood specimen from the specimen container T using the syringe pump (not shown). The specimen thus suctioned is transferred to the position of the first mixing chamber MC1 and the second mixing chamber MC2, and a predetermined amount of the whole blood specimen is dispensed to each of the chambers MC1 and MC2 using the syringe pump.

The sample preparation portion 220 is connected via a tube with a reagent container 221a for housing a first reagent, a reagent container 221b for housing a second reagent, a reagent container 222a for housing a third reagent, a reagent container 222b for housing a fourth reagent, and a reagent container 223 for housing a sheath fluid (diluting fluid). The sample preparation portion 220 is also connected with a compressor, and the respective reagents can be drawn from the corresponding reagent containers 221a, 221b, 222a, 222b, and 223 with the pressure generated by the compressor.

The third reagent is a hemolyzing agent for measuring nucleated erythrocytes (NRBCs). Examples of this hemolyzing agent for measuring NRBCs include a Stromatolyser NR Lyse manufactured by Sysmex Corporation. The fourth reagent is a stain solution containing a fluorescent dye for measuring NRBCs. Examples of this stain solution for measuring NRBCs include a Stromatolyser NR Dye manufactured by Sysmex Corporation.

Note that the first reagent, the second reagent and the sheath fluid are the same as those in Embodiment 1, and therefore the description thereof is omitted.

Furthermore, since configurations of other elements of the blood analyzer 200 according to this embodiment are the same as those of the blood analyzer 1 according to Embodiment 1, the same elements are denoted by the same reference numerals, and the descriptions thereof are omitted.

### Operation of Blood Analyzer

Next, the specimen measuring operation of the blood analyzer 200 according to this embodiment will be described.

The blood analyzer 200 according to this embodiment can perform an abnormal lymphocyte/blast measurement and an NRBC measurement using the optical detector D. The measurement steps include a first measurement step in which a measurement sample for abnormal lymphocyte/blast is measured, a second measurement step in which a measurement sample for NRBC is measured, and a data processing step in which the measurement data obtained in the first measurement step and the second measurement step are subjected to analysis processing.

First, the sample rack L holding the specimen container T is placed on the specimen carrying unit 4 by the operator. The sample rack L is carried by the specimen carrying unit 4, and the specimen container T housing a specimen to be measured is positioned in the specimen supply position 43a. Next, the specimen container T is gripped by the hand portion 25a of the measurement unit 202, and the specimen container T is taken out from the sample rack L. The hand portion 25a then makes an oscillating movement, and thereby the specimen inside the specimen container T is agitated. Next, the specimen container T is inserted into the specimen container setting portion 25b, and the specimen container setting portion 25b is moved in the Y2 direction. After the bar code of the specimen is read by the bar code reading portion 26, the specimen container T reaches the suction position. Then, the first measurement step and the second measurement step are performed.

In the first measurement step, a measurement sample for abnormal lymphocytes/blasts is prepared by supplying a predetermined amount of the first reagent, a predetermined amount of the second reagent, and a predetermined amount of the whole blood specimen to the first mixing chamber MC1, and optical measurement is conducted on the measurement sample for abnormal lymphocytes/blasts with the optical detector D. Since this first measurement step is the same as the measurement step according to Embodiment 1, the detailed description thereof is omitted. In the first measurement step, a first measurement data which is a digital data containing feature parameters of a forward scattered light signal (forward scattered light intensity), a side scattered light signal (side scattered light intensity), and a fluorescence signal (fluorescence intensity) is generated, and the first measurement data is transmitted to the information processing unit 5.

### Second Measurement Step

Next, the second measurement step will be described. The second measurement step is performed in such a manner that it partially overlaps in time with the first measurement step. In the second measurement step, the blood analyzer 200 mixes the whole blood specimen (17.0 µL), the third reagent (1.0 mL), and the fourth reagent (0.030 mL) to prepare an NRBC measurement sample, and measures the NRBC measurement sample by flow cytometry using the optical detector D.

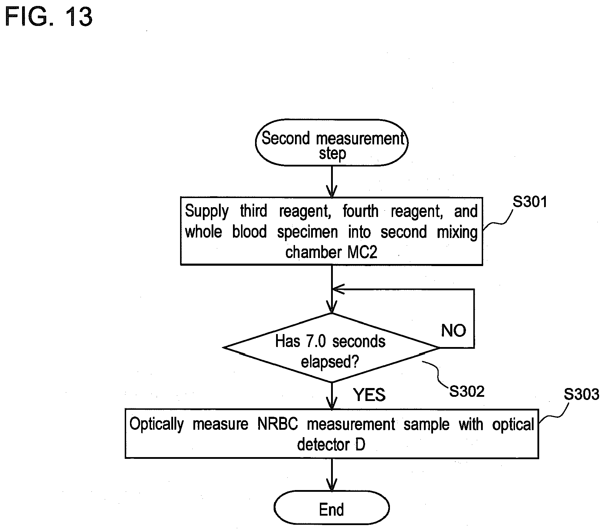

FIG. 13 is a flowchart illustrating the procedure of operation performed by the blood analyzer 200 in the second measurement step. The CPU 51a controls the measurement unit 202 to supply, to the second mixing chamber MC2, the third reagent (1.0 mL) from the reagent container 222a, the fourth reagent (0.030 mL) from the reagent container 222b, and the whole blood specimen (17.0 µL) from the suction tube 211 (step S301). In step S301, the specimen supplied to the second mixing chamber MC2 is a portion of the whole blood specimen suctioned by the suction tube 211 in the first measurement step described above. In other words, in the first measurement step, the specimen to be supplied to the first mixing chamber MC1 and the specimen to be supplied to the second mixing chamber MC2 are suctioned at a time from the specimen container T.

Next, the CPU 51a waits 7.0 seconds and determines whether 7.0 seconds have elapsed since the supply of the third reagent, the fourth reagent and the whole blood specimen to the second mixing chamber MC2 (step S302). Here, the second mixing chamber MC2 has been heated to 41.0°C by the heater. Thus, the mixed solution of the third reagent, the fourth reagent and the blood specimen is heated at 41.0°C for 7.0 seconds to prepare an NRBC measurement sample.

Then, optical measurement is conducted on the NRBC measurement sample with the optical detector D (step S303). Specifically, in the processing of step S303, the NRBC measurement sample and the sheath fluid are simultaneously supplied to the flow cell 231 of the optical detector D. At that time, forward scattered light is received by the photodiode 243, and side scattered light is received by the photodiode 246, and fluorescence is received by the avalanche photodiode 248. Output signals (analog signals) output from these various light-receiving elements of the optical detector D are converted into digital signals as in the first measurement step (measurement step according to Embodiment 1) described above, and then converted into second measurement data that is digital data through predetermined signal processing. The second measurement data is transmitted to the information processing unit 5. In this signal processing, a forward scattered light signal (forward scattered light intensity), a side scattered light signal (side scattered light intensity), and a fluorescence signal (fluorescence intensity) are obtained as feature parameters contained in the second measurement data. This completes the second measurement step. As will be described later, the CPU 51a of the information processing unit 5 performs predetermined analysis processing on the second measurement data, thereby generating analysis result data containing the numeric data of NRBCs, and stores the analysis result data in the hard disk 51d.

### Data Processing Step

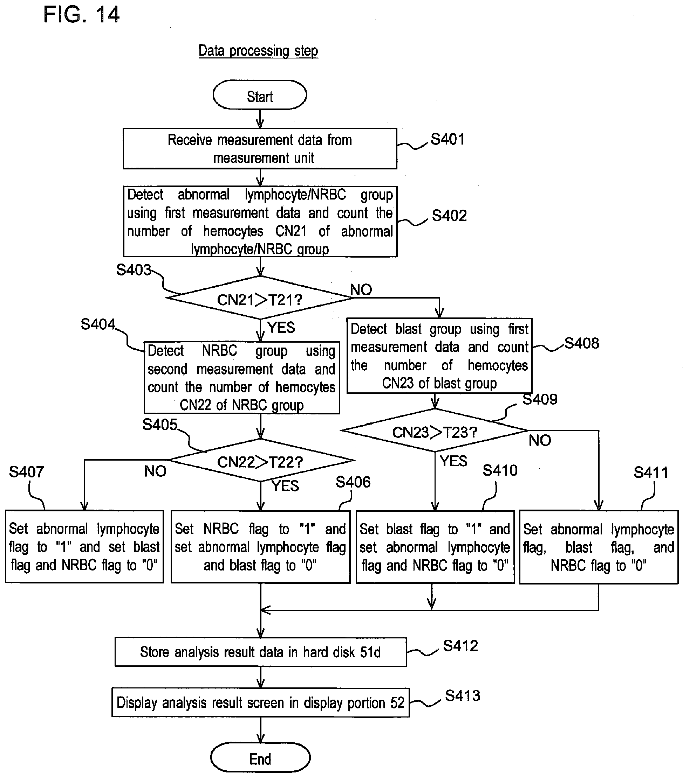

Next, the data processing step will be described. FIG. 14 is a flowchart showing the procedure of processing in a data processing step performed by the blood analyzer according to this embodiment. The information processing unit 5 of the blood analyzer 200 receives the measurement data from the measurement unit 202 (step S401). The computer program 54a, which is executed by the CPU 51a, is an event-driven program, and the processing of step S402 is invoked upon occurrence of an event of receiving the measurement data.

In step S402, the CPU 51a detects a cell group of abnormal lymphocytes or NRBCs (hereinafter, referred to as an "abnormal lymphocyte/NRBC group") using the first measurement data, and counts the number of hemocytes CN21 contained in the detected abnormal lymphocyte/NRBC group (step S402).

In this embodiment, the detection area A1 in FIG. 7 is defined as the range of forward scattered light intensity and fluorescence intensity for detecting the abnormal lymphocyte/NRBC group. As a result of experiments using clinical specimens and detailed investigation of the results of the experiments, the present inventors have found that NRBCs as well as abnormal lymphocytes appear in the detection area A1 and the area A11 shown in FIG. 8. That is to say, when a hemocyte group is detected in the area A1 shown in FIG. 7 or in the area A11 shown in FIG. 8, there is a case where the hemocyte group is not the abnormal lymphocyte group, but is a cell group of NRBCs (hereinafter, referred to as an "NRBC group,"). Note that in step S402, processing for detecting an abnormal lymphocyte/NRBC group is the same as that for detecting an abnormal lymphocyte group in step S202 of Embodiment1, and therefore the detailed description thereof is omitted. In this embodiment, when a hemocyte group is detected in the detection area A1, it is determined, using the second measurement data, that the hemocyte group is an abnormal lymphocyte group or an NRBC group.

Next, the CPU 51a determines whether CN21 is greater than a predetermined threshold T21 (step S403). The threshold T21 is a reference value for determining whether any abnormal lymphocyte or any NRBCs is present in a blood specimen. In step S403, if CN21 is greater than the threshold T21, it is determined that abnormal lymphocytes or NRBCs are present in the blood specimen. If CN21 is less than or equal to the threshold T21, it is determined that neither abnormal lymphocytes nor NRBCs are present in the blood specimen.

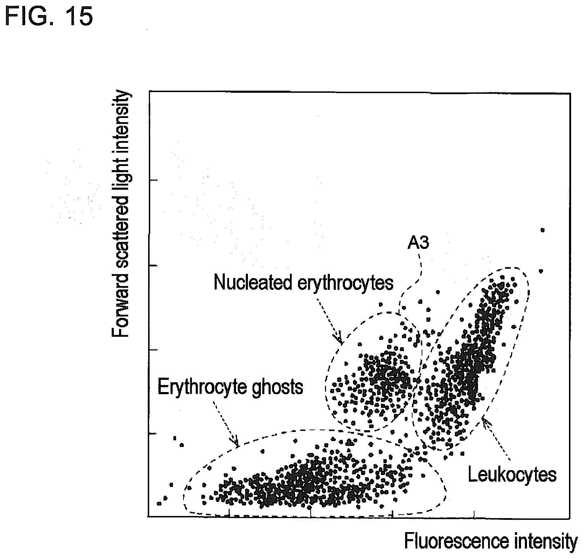

If CN21 > T21 in step S403 (YES in step S403), the CPU 51a classifies the NRBC group and other hemocyte groups using the second measurement data and counts the number of nucleated erythrocytes CN22 (step S404). The processing will be described in detail. FIG. 15 is a scattergram of forward scattered light intensity and fluorescence intensity in second measurement data. A cluster of nucleated erythrocytes, a cluster of leukocytes and a cluster of erythrocyte ghosts appear in the scattergram shown in FIG. 15. In this embodiment, the range of forward scattered light intensity and fluorescence intensity indicated by the area A3 in FIG. 15 is defined as a detection area for an NRBC group. As shown in this scattergram, the area A3 can be used by utilizing forward scattered light intensity and fluorescence intensity of the second measurement data to distinguish the cluster of nucleated erythrocytes from other clusters. In a processing of step S404, the CPU 51a distinguish nucleated erythrocytes from other clusters using forward scattered light intensity and fluorescence intensity of the second measurement data, and thereby detects an NRBC group. Moreover, the CPU 51a counts the number of detected nucleated erythrocytes CN22. Note that, for example, the number of NRBCs per 1µL of blood or the number of NRBCs per 100 leukocytes can be used as the number of hemocytes of an NRBC group CN22.

Next, the CPU 51a determines whether CN22 is greater than a predetermined threshold T22 (step S405). The threshold T22 is a reference value for determining whether any NRBC is present in a blood specimen. In step S405, if CN22 is greater than the threshold T22, it is determined that NRBCs are present in the blood specimen. If CN22 is less than or equal to the threshold T22, it is determined that no NRBCs are present in the blood specimen.

If CN22 > T22 in step S405 (YES in step S405), the CPU 51a sets the NRBC flag provided in the RAM 51c to "1", and sets each of the abnormal lymphocyte flag and the blast flag to "0" (step S406). Here, the NRBC flag is a flag indicating the presence or absence of NRBCs in a blood specimen. The NRBC flag indicates the presence of NRBCs if it is set to "1", and indicates the absence of NRBCs if it is set to "0". Note that the abnormal lymphocyte flag and blast flag are the same as in Embodiment 1, and therefore the descriptions thereof are omitted. Then, the processing executed by the CPU 51a moves to step S412.

On the other hand, if CN22 ≤ T22 in step S405 (NO in step S405), it is determined that no NRBCs are present in the blood specimen. That is, in step S402, it is established that a hemocyte group detected in the detection area A1 is an abnormal lymphocyte group. Accordingly, in this case, the CPU 51a sets the abnormal lymphocyte flag provided in the RAM 51c to "1", and sets each of the NRBC flag and the blast flag to "0" (step S407). Then, the processing executed by the CPU 51a moves to step S412.

If CN21 ≤ T21 in step S403 (NO in step S403), the CPU 51a detects a blast group using the first measurement data, and counts the number of hemocyte CN23 contained in the detected blasts group (step S408). Note that the processing of step S408 is the same as that of step S205 described in Embodiment 1, and therefore the description thereof is omitted.

Next, the CPU 51a determines whether CN23 is greater than a predetermined threshold T23 (step S409). The threshold T23 is a reference value for determining whether any blast is present in a blood specimen. In step S409, if CN23 is greater than the threshold T23, it is determined that blasts are present in the blood specimen. If CN23 is less than or equal to the threshold T23, it is determined that no blasts are present in the blood specimen.

If CN23 > T23 (YES in step S409), the CPU 51a sets the blast flag provided in the RAM 51c to "1", and sets each of the abnormal lymphocyte flag and the NRBC flag to "0" (step S410). Then, the processing executed by the CPU 51a moves to step S412.

On the other hand, if CN23 ≤ T23 (NO in step S409), the CPU 51a sets each of the abnormal lymphocyte flag, the blast flag and the NRBC flag provided in the RAM 51c to "0" (step S411). Then, the processing executed by the CPU 51a moves to step S412.

In step S412, the CPU 51a stores the thus obtained analysis result (including the abnormal lymphocyte flag, the blast flag and the NRBC flag) in the hard disk 51d. Next, the CPU 51a causes the image display portion 52 to display an analysis result screen showing the analysis result stored in the hard disk 51d (step S413), and ends the processing.

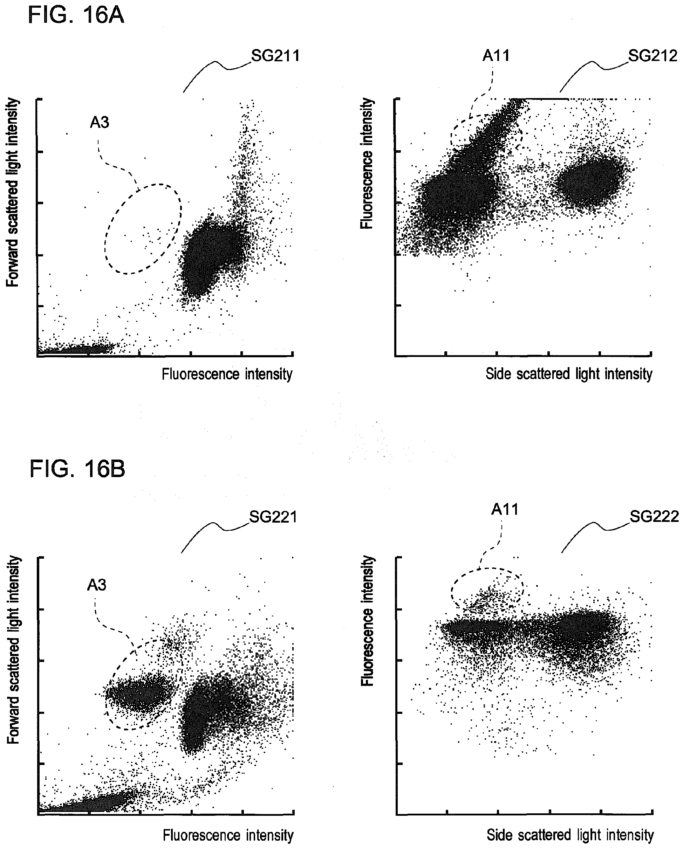

Next, examples of scattergrams obtained when measuring a specific blood specimen are shown, and the analysis of the measurement data performed by the blood analyzer 200 according to this embodiment will be described. FIG. 16A and FIG. 16B show examples of scattergrams obtained in substantially the same measurement condition as the measurement condition by the blood analyzer 200. FIG. 16A shows examples of scattergrams obtained when measuring a blood specimen F containing abnormal lymphocytes. FIG. 16B shows examples of scattergrams obtained when measuring a blood specimen G containing NRBCs.

As shown in FIG. 16A, hemocytes are substantially absent (they are present in a very small amount, but the number of hemocytes does not exceed the threshold T22) in the detection area A3 for NRBCs in a scattergram SG211 of forward scattered light intensity and fluorescence intensity (scattergram for NRBC detection) in the second measurement data of the blood specimen F. FIG. 16A also shows a scattergram SG212 of fluorescence intensity and side scattered light intensity in the first measurement data of the blood specimen F. In the scattergram SG212, hemocytes are present in the area A11 where abnormal lymphocytes or NRBCs appear. That is, although the abnormal lymphocyte/NRBC group is detected in the blood specimen F as can be seen from the scattergram SG212, the NRBC group is not detected in the blood specimen F as can be seen from the scattergram SG211. Accordingly, it is established that the hemocyte group that appears in the area A11 is an abnormal lymphocyte group. Thus, CN21 > T21 and CN22 ≤ T22 when the blood analyzer 200 according to this embodiment analyses the blood specimen F, and therefore it is determined that abnormal lymphocytes are present.

As shown in FIG. 16B, hemocytes are present in the detection area A3 for the NRBCs in a scattergram SG221 of forward scattered light intensity and fluorescence intensity (scattergram for NRBC detection) in the second measurement data of the blood specimen G. FIG. 16B also shows a scattergram SG222 of fluorescence intensity and side scattered light intensity in the first measurement data of the blood specimen G. In the scattergram SG222, hemocytes are present in the area A11 where abnormal lymphocytes or NRBCs appear. That is, the abnormal lymphocyte/NRBC group is detected in the blood specimen G as can be seen from the scattergram SG222, and the NRBC group is also detected in the blood specimen G as can be seen from the scattergram SG221. Accordingly, it is established that the hemocyte group that appears in the area A11 is an NRBC group. Thus, CN21 > T21 and CN22 > T22 when the blood analyzer 200 according to this embodiment analyses the blood specimen G, and therefore it is determined that NRBCs are present.

As described above, when a blood specimen containing abnormal lymphocytes and containing none of blasts, NRBCs and atypical lymphocytes is measured by the blood analyzer 200, abnormal lymphocytes are detected, and blasts and NRBCs are not detected. Similarly, when a blood specimen containing NRBCs and containing none of abnormal lymphocytes, blasts and atypical lymphocytes is measured by the blood analyzer 200, NRBCs are detected, and neither abnormal lymphocytes nor blasts are detected. Similarly, when a blood specimen containing blasts and containing none of abnormal lymphocytes, NRBCs and atypical lymphocytes is measured by the blood analyzer 200, blasts are detected, and neither abnormal lymphocytes nor NRBCs are detected. Furthermore, when a blood specimen containing atypical lymphocytes and containing none of abnormal lymphocytes, blasts and NRBCs is measured, neither abnormal lymphocytes, blasts nor NRBCs are detected. That is, with the blood analyzer 200 according to the embodiment, it is possible to detect abnormal lymphocytes, blasts and NRBCs separately.

Although not shown, when the NRBC flag is set to "1" and each of the abnormal lymphocyte flag and the blast flag is set to "0", "NRBC present" is indicated in the flag indication field FLG on the analysis result screen shown in FIG. 11. When the abnormal lymphocyte flag is set to "1" and each of the NRBC flag and the blast flag is set to "0", "Abn Lympho?" is indicated, as shown in FIG. 11, in the flag indication field FLG on the analysis result screen. When the blast flag is set to "1" and each of the abnormal lymphocyte flag and the NRBC flag is set to "0", "Blasts?" is indicated in the flag indication field FLG. Furthermore, when each of the abnormal lymphocyte flag, the blast flag, and the NRBC flag is set to "0", none of "NRBC present", "Abn Lympho?" and "Blasts?" above will be indicated on the analysis result screen. This enables the operator to understand whether any abnormal lymphocyte is detected in the blood specimen, whether any blast is detected in the blood specimen, and whether any NRBC is detected in the blood specimen simply by looking at the analysis result screen.

With the configuration described above, the blood analyzer 200 can detect abnormal lymphocytes, blasts and NRBCs separately by measuring, with the optical detector D, an abnormal lymphocyte/blast measurement sample prepared by mixing the blood specimen, the first reagent containing a hemolyzing agent, and the second reagent containing a fluorescent dye for staining nucleic acid, and measuring, with the optical detector D, an NRBC measurement sample prepared by mixing the blood specimen, the third reagent, and the fourth reagent. Furthermore, the blood analyzer 200 can suppress misdetections of NRBCs as abnormal lymphocytes.

### Other Embodiments

Note that the reaction temperature and the reaction time during mixing of the blood specimen, the first reagent, and the second reagent in the sample preparation portion 22 may be suitably set according to the state of damage or staining of the hemocytes contained in the blood specimen, without any particular limitation. Specifically, the reaction time and the reaction temperature may be adjusted such that the reaction time is short when the reaction temperature is high and the reaction time is long when the reaction temperature is low. More specifically, it is preferable that the blood specimen and the reagents are mixed at a temperature of 20°C to 45°C for 3 to 40 seconds.

Although the above-described embodiment has addressed a configuration in which the first reagent containing a hemolyzing agent and the second reagent containing a fluorescent dye that can stain nucleic acid are used to perform the measurement step, the present invention is not limited thereto. It is possible to adopt a configuration in which the measurement sample is prepared by mixing the blood specimen with a single reagent containing a hemolyzing agent and a nucleic acid staining dye, and abnormal lymphocytes and blasts are detected using the measurement sample. In this case, the concentrations of the surfactant and the fluorescent dye are adjusted to the above-described concentrations when the reagents have been mixed.

Although the above-described embodiment has addressed a configuration in which the presence or absence of abnormal lymphocytes in the blood specimen is determined based on whether the number of hemocytes CN1 that appeared within the detection area A1 is greater than the threshold T1 for abnormal lymphocytes with respect to forward scattered light intensity and fluorescence intensity in the measurement data, the present invention is not limited thereto. It is also possible to adopt a configuration in which the ratio of the number of hemocytes that appeared within the detection area A1 for abnormal lymphocytes to the total number of leukocytes is obtained, and whether abnormal lymphocytes are present is determined by determining whether the obtained ratio is greater than a predetermined reference value. Likewise, for detection of blasts as well, it is also possible to adopt a configuration in which the ratio of the number of hemocytes that appeared within the detection area A2 for blasts to the total number of leukocytes is obtained, and whether any blast is present is determined by determining whether the obtained ratio is greater than a predetermined reference value.

Although the above-described embodiment has addressed a configuration in which the measurement sample is optically measured by flow cytometry to obtain an optical signal including fluorescence intensity, forward scattered light intensity, and side scattered light intensity, and the fluorescence intensity and the scattered light intensity are used to detect a cell group not containing blasts and atypical lymphocyte, but containing abnormal lymphocytes in a blood specimen, the present invention is not limited thereto. It is also possible to adopt a configuration in which scattered light information other than forward scattered light intensity and side scattered light intensity, such as wide angle forward scattered light intensity, is obtained along with fluorescence intensity, and a cell group not containing blasts and atypical lymphocytes, but containing abnormal lymphocytes is detected in the predetermined range of the scattered light information and the fluorescence intensity

Although the above-described embodiment has addressed a configuration in which the control of the measurement unit 2 and the processing of the measurement data are performed by the CPU 51a executing the above-described computer program 54a, the present invention is not limited thereto. It is also possible to adopt a configuration in which the control of the measurement unit 2 and the processing of measurement data are performed by dedicated hardware, such as FPGA or ASIC, that can perform the same processing as that performed by the computer program 54a.

Although the above-described embodiment has addressed a configuration in which a single computer 5a executes all the processing of the computer program 54a, the present invention is not limited thereto. It is also possible to adopt a distributed system in which the same processing as that of the above-described computer program 54a is executed by a plurality of devices (computers) in a distributed manner.

In Embodiment 2 described above, although the CPU 51a determines that a hemocyte group that appeared in the detection area A1 is an NRBC group if CN22 > T22 in S405 of the data processing step, the CPU 51a may further perform steps for determining an appearance of blasts (S408 and S409) if CN22 >T22 in S405. Both NRBCs and blasts are blood cells which are normally present in bone marrow. Therefore, both NRBCs and blasts may appear in peripheral blood due to bone marrow diseases.

In Embodiment 2 described above, although the CPU 51a determines whether NRBCs are present, and whether abnormal lymphocytes are present, in the data processing step, the CPU 51a may perform the step for determining whether NRBCs are present independently of the step for determining whether abnormal lymphocytes are present.

The same value as the threshold T21 that is used to determine whether abnormal lymphocytes are present, or the different value from the T21 may be used as the threshold T22 that is used to determine whether NRBCs are present.

### Performance Evaluation Experiment

The present inventors conducted a performance evaluation experiment of a blood analysis method performed with the above-described blood analyzer 1.

In this experiment, a plurality of blood specimens was examined with a microscope by hand method, and the numbers of lymphoblasts, abnormal lymphocytes and atypical lymphocytes were counted. If the ratio of the number of lymphoblasts to the number of total leukocytes was not less than 2%, the blood specimen was determined to be "positive" for lymphoblasts. If the ratio of the number of abnormal lymphocytes to the number of total leukocytes was not less than 10%, the blood specimen was determined to be "positive" for abnormal lymphocytes. If the ratio of the number of atypical lymphocytes to the number of total leukocytes was not less than 2%, the blood specimen was determined to be "positive" for atypical lymphocytes. Furthermore, the blood specimen in which no appearances of lymphoblasts, abnormal lymphocytes and atypical lymphocytes were observed was determined to be a "negative specimen".

In this experiment, the specimen that was determined to be positive for lymphoblasts by the above-described microscopic examination and was not determined to be positive for abnormal lymphocytes and atypical lymphocytes (hereinafter, referred to as a "lymphoblast positive specimen") was measured by flow cytometry by following the procedure below. Likewise, in this experiment, the specimen that was determined to be positive for abnormal lymphocytes by the microscopic examination and was not determined to be positive for lymphoblasts and atypical lymphocytes (hereinafter, referred to as an "abnormal lymphocyte positive specimen") was measured by flow cytometry. The specimen that was determined to be positive for atypical lymphocytes by the microscopic examination and was not determined to be positive for lymphoblasts and abnormal lymphocytes (hereinafter, referred to as an "atypical lymphocyte positive specimen") was also measured by following the procedure below. Furthermore, the above-described "negative specimen" was measured by flow cytometry.

The following reagents were used for the measurement by flow cytometry.

### Hemolyzing Agent

MOPS: 2.09 g/L
polyoxyethylene (20) oleyl ether: 1.25 g/L
sodium N-lauroylsarcosinate: 0.268 g/L
EDTA-2K: 0.5 g/L

The above-listed ingredients were mixed, and NaOH was further added to adjust the pH to 7.3. The osmotic pressure of the first reagent was 37 mOsm/Kg, and the electric conductivity thereof was 0.745 mS/cm.

### Staining Reagent

NK-321: 50 mg/L
NK-321 (50 mg/L) dissolved in ethylene glycol was used as the second reagent.

### Measurement

A measurement sample was prepared by mixing a whole blood specimen (17.0 µL), a hemolyzing agent (1000 µL), and a staining reagent (20 µL), and measured by flow cytometry using the optical detector. A red semiconductor laser having an excitation wavelength of 633 nm was used as a light source to detect fluorescence (red fluorescence) having a wavelength of 650 nm or more as a fluorescence signal.

In the scattergram of forward scattered light intensity and fluorescence intensity obtained by performing the measurement on each specimen, the number of hemocytes that appeared in the area corresponding to the detection area A1 shown in FIG. 7 (area for detecting abnormal lymphocytes) was counted, and the ratio HLF% of the number of hemocytes to the number of the total leukocytes was found. If the ratio HLF% was not less than a reference value (1%), the specimen was determined to be positive for abnormal lymphocytes, and if the ratio HLF% was less than the reference value, the specimen was determined to be negative for abnormal lymphocytes.

In the scattergram of forward scattered light intensity and side scattered light intensity obtained by measuring the specimens, the number of hemocytes LC1# that appeared in the area corresponding to the detection area A2 shown in FIG. 9 (area for detecting blasts) was counted. If the number of hemocytes LC1# was not less than a reference value (20), the specimen was determined to be positive for blasts, and if the number of hemocytes LC1# was less than the reference value, the specimen was determined to be negative for blasts.

Experimental results on the lymphoblast positive specimen are shown in Table 1. Note that values in Table 1 show the number of specimens (similarly in Tables 2 to 4).

**Table 1**

| | | Lymphoblast positive specimen |
|---|---|---|
| Determination result of abnormal lymphocyte | Positive | 0 (0.0%) |
| | Negative | 11 |
| Determination result of blast | Positive | 9 (81.8%) |
| | Negative | 2 |

As a result of measuring all of 11 lymphoblast positive specimens by flow cytometry, no specimen was determined to be positive for abnormal lymphocytes. That is to say, the number of specimens in which abnormal lymphocytes were detected by flow cytometry measurement is 0, and the number of specimens in which no abnormal lymphocytes were detected was 11, among the lymphoblast positive specimens. As a result of flow cytometry measurement, the number of specimens that was determined to be positive for blasts was 9, and the number of specimens that was determined to be negative for blasts was 2, among the 11 lymphoblast positive specimens. That is to say, blasts were detected in 81.8% of the lymphoblast positive specimens by flow cytometry measurement. This shows that lymphoblasts contained in a lymphoblast positive specimen do not substantially appear in the detection area A1 for detecting abnormal lymphocytes, and appear in the detection area A2 for detecting blasts. That is, with the blood analysis method according to the embodiment, it is possible to accurately detect blasts in a blood specimen containing lymphoblasts, and not containing abnormal lymphocytes and atypical lymphocytes without misdetection of abnormal lymphocytes.

Experimental results on the abnormal lymphocyte positive specimen are shown in Table 2.

**Table 2**

| | | Abnormal lymphocyte positive specimen |
|---|---|---|
| Determination result of abnormal lymphocyte | Positive | 8 (66.7%) |
| | Negative | 4 |
| Determination result of blast | Positive | 0 (0.0%) |
| | Negative | 12 |

As a result of flow cytometry measurement, the number of specimens that was determined to be positive for abnormal lymphocytes was 8, and the number of specimens that was determined to be negative for abnormal lymphocytes was 4, among the 12 abnormal lymphocyte positive specimens. That is to say, abnormal lymphocytes were detected in 66.7% of the abnormal lymphocyte positive specimens by flow cytometry measurement. As a result of measuring all of the 12 abnormal lymphocyte positive specimens by flow cytometry, no specimen was determined to be positive for blasts. That is to say, the number of specimens in which blasts were detected by flow cytometry measurement is 0, and the number of specimens in which no blasts were detected was 12, among the abnormal lymphocyte positive specimens. This shows that abnormal lymphocytes contained in an abnormal lymphocyte positive specimen appear in the detection area A1 for detecting abnormal lymphocytes, and do not substantially appear in the detection area A2 for detecting blasts. That is, with the blood analysis method according to the embodiment, it is possible to accurately detect abnormal lymphocytes in a blood specimen containing abnormal lymphocytes, and not containing lymphoblasts and atypical lymphocytes without misdetection of blasts.

Experimental results on the atypical lymphocyte positive specimen are shown in Table 3.

**Table 3**

| | | Atypical lymphocyte positive specimen |
|---|---|---|
| Determination result of abnormal lymphocyte | Positive | 2 (5.9%) |
| | Negative | 32 |
| Determination result of blast | Positive | 0 (0.0%) |
| | Negative | 34 |

As a result of flow cytometry measurement, the number of specimens that was determined to be positive for abnormal lymphocytes was 2, and the number of specimens that was determined to be negative for abnormal lymphocytes was 32, among the 34 atypical lymphocyte positive specimens. That is to say, abnormal lymphocytes were not detected in almost all of the atypical lymphocyte positive specimens by flow cytometry measurement. As a result of measuring all of 34 atypical lymphocyte positive specimens by flow cytometry, no specimen was determined to be positive for blasts. That is to say, the number of specimens in which blasts were detected by flow cytometry measurement is 0, and the number of specimens in which no blasts were detected was 34, among the atypical lymphocyte positive specimens. This shows that atypical lymphocytes contained in an atypical lymphocyte positive specimen do not substantially appear in the detection area A1 for detecting abnormal lymphocytes and in the detection area A2 for detecting blasts. That is, with the blood analysis method according to the embodiment, blasts and abnormal lymphocytes are rarely misdetected in a blood specimen containing atypical lymphocytes, and not containing lymphoblasts and abnormal lymphocytes.

Experimental results on the negative specimen are shown in Table 4.

**Table 4**

| | | Negative specimen |
|---|---|---|
| Determination result of abnormal lymphocyte | Positive | 11 (0.9%) |
| | Negative | 1168 |
| Determination result of blast | Positive | 4 (0.3%) |
| | Negative | 1175 |

As a result of flow cytometry measurement, the number of specimens that was determined to be positive for abnormal lymphocytes was 11, and the number of specimens that was determined to be negative for abnormal lymphocytes was 1168, among the 1179 negative specimens. That is to say, abnormal lymphocytes were not detected in almost all of the negative specimens by flow cytometry measurement (where abnormal lymphocytes were detected in 0.9% of all of the negative specimens). As a result of flow cytometry measurement, the number of specimens that was determined to be positive for blasts was 4, and the number of specimens that was determined to be negative for blasts was 1175, among the 1179 negative specimens. That is to say, blasts were not detected in almost all of the negative specimens by flow cytometry measurement (where blasts were detected in 0.3% of all of the negative specimens). This shows that atypical lymphocytes contained in an atypical lymphocyte positive specimen do not substantially appear in the detection area A1 for detecting abnormal lymphocytes and in the detection area A2 for detecting blasts. That is, with the blood analysis method according to the embodiment, blasts and abnormal lymphocytes are rarely misdetected in a blood specimen containing none of atypical lymphocytes, lymphoblasts and abnormal lymphocytes.

### Industrial Applicability

A blood analyzer, a blood analysis method and a computer program of the present invention are useful as a blood analyzer and a blood analysis method for optically measuring a blood specimen and detecting a cell group contained in the blood specimen, and a computer program for enabling a computer to analyze blood.

Further embodiments are described as E1 to E19 as follows:
E1. A blood analyzer comprising a sample preparation portion for preparing a measurement sample by mixing a hemolyzing agent not substantially containing a cationic surfactant, but containing a nonionic surfactant, a blood specimen, and a fluorescent dye for staining nucleic acid; a light source for irradiating the measurement sample prepared by the sample preparation portion with light; a light-receiving portion for receiving fluorescence and scattered light that are produced by cells in the measurement sample when the light source irradiates the measurement sample with light to output a fluorescence signal relating to the received fluorescence and a scattered light signal relating to the received scattered light; a cell analysis portion for detecting cells showing fluorescence intensity and scattered light intensity in a predetermined range as abnormal lymphocytes based on the fluorescence signal and the scattered light signal output by the light-receiving portion; and an output portion for performing output based on a result of a detection made by the cell analysis portion.
E2. The blood analyzer according to E1, wherein the cell analysis portion detects the cells showing fluorescence intensity and scattered light intensity in the predetermined range as abnormal lymphocytes distinguishably from blasts and atypical lymphocytes.
E3. The blood analyzer according to E1 or E2, wherein the light-receiving portion is configured to receive forward scattered light and side scattered light produced by cells in the measurement sample when the measurement sample is irradiated with light to output the scattered light signal containing a forward scattered light signal relating to the received forward scattered light and a side scattered light signal relating to the received side scattered light, and the cell analysis portion is configured to detect cells showing fluorescence intensity and scattered light intensity in a predetermined range as abnormal lymphocytes based on the fluorescence signal and at least one of the forward scattering light signal and the side scattering light signal.
E4. The blood analyzer according to any one of E1 to E3, wherein the light-receiving portion is configured to receive forward scattered light and side scattered light produced by cells in the measurement sample when the measurement sample is irradiated with light to output the scattered light signal containing a forward scattered light signal relating to the received forward scattered light and a side scattered light signal relating to the received side scattered light, and the cell analysis portion is configured to detect blasts distinguishably from abnormal lymphocytes and atypical lymphocytes based on at least two signals selected from the forward scattered light signal, the side scattered light signal, and the fluorescence signal.
E5. The blood analyzer according to E4, wherein the cell analysis portion is configured to detect cells showing forward scattered light intensity and side scattered light intensity in a predetermined range as blasts distinguishably from abnormal lymphocytes and atypical lymphocytes based on the forward scattered light signal and the side scattered light signal.
E6. The blood analyzer according to any one of E1 to E5, wherein the sample preparation portion is configured to prepare a measurement sample by mixing the hemolyzing agent not substantially containing a cationic surfactant, but containing a nonionic surfactant and an anionic surfactant, the blood specimen, and the fluorescent dye.
E7. The blood analyzer according to any one of E1 to E6, wherein the sample preparation portion is configured to prepare a second measurement sample by mixing a second hemolyzing agent for measuring nucleated erythrocytes, a blood specimen, and a second fluorescent dye for measuring nucleated erythrocytes, the light-receiving portion is configured to receive fluorescence and scattered light produced by cells in the second measurement sample when the light source irradiates the second measurement sample with light to output a second fluorescence signal relating to the received fluorescence and a second scattered light signal relating to the received scattered light, and the cell analysis portion is configured to perform a detection process for detecting nucleated erythrocytes in the second measurement sample based on the second fluorescence signal and the second scattered light signal output by the light-receiving portion, and detect the cells showing fluorescence intensity and scattered light intensity in the predetermined range as abnormal lymphocytes if a result of the detection process satisfies a predetermined condition.
E8. A blood analyzer comprising a sample preparation portion for preparing a first measurement sample from a blood specimen and a first reagent, and a second measurement sample from a blood specimen and a second reagent; a light source for irradiating the first measurement sample and the second measurement sample prepared by the sample preparation portion with light; a light-receiving portion for receiving fluorescence and scattered light that are produced by cells in the first measurement sample when the light source irradiates the first measurement sample with light to output a first fluorescence signal relating to the received fluorescence and a first scattered light signal relating to the received scattered light, and receiving fluorescence and scattered light that are produced by cells in the second measurement sample when the light source irradiates the first measurement sample with light to output a second fluorescence signal relating to the received fluorescence and a second scattered light signal relating to the received scattered light; a cell analysis portion for performing a detection process for detecting nucleated erythrocytes in the second measurement sample based on the second fluorescence signal and the second scattered light signal, and detecting cells showing fluorescence intensity and scattered light intensity in a predetermined range as abnormal lymphocytes based on the first fluorescence signal and the first scattered light signal if a result of the detection process satisfies a predetermined condition; and an output portion for performing output based on a result of a detection made by the cell analysis portion.
E9. The blood analyzer according to E8, wherein the cell analysis portion performs an obtaining process, as the detection process, for obtaining values based on the number of cells showing fluorescence intensity and scattered light intensity in a second predetermined range on the basis of the second fluorescence signal and the second scattered light signal, and detects the cells showing fluorescence intensity and scattered light intensity in the predetermined range as abnormal lymphocytes if the obtained value is not greater than a predetermined threshold.
E10. A blood analysis method comprising steps of preparing a measurement sample by mixing a hemolyzing agent not substantially containing a cationic surfactant, but containing a nonionic surfactant, a blood specimen, and a fluorescent dye for staining nucleic acid; irradiating the measurement sample prepared with light; receiving fluorescence and scattered light that are produced by cells in the measurement sample when the measurement sample is irradiated with light to obtain a fluorescence signal relating to the received fluorescence and a scattered light signal relating to the received scattered light; detecting cells showing fluorescence intensity and scattered light intensity in a predetermined range as abnormal lymphocytes based on the fluorescence signal and the scattered light signal; and performing output based on a result of a detection.
E11. The blood analysis method according to E10, wherein in the step of detecting abnormal lymphocytes, the cells showing fluorescence intensity and scattered light intensity in the predetermined range are detected as abnormal lymphocytes distinguishably from blasts and atypical lymphocytes.
E12. The blood analysis method according to E10 or E11, wherein in the step of receiving the fluorescence signal and the scattered light signal, fluorescence, forward scattered light, and side scattered light that are produced by cells in the measurement sample are received when the measurement sample is irradiated with light to obtain the fluorescence signal relating to the received fluorescence and the scattered light signal containing a forward scattered light signal relating to the received forward scattered light and a side scattered light signal relating to the received side scattered light, and in the step of detecting abnormal lymphocytes, cells showing fluorescence intensity and scattered light intensity in a predetermined range are detected as abnormal lymphocytes based on the fluorescence signal and at least one of the forward scattering light signal and the side scattering light signal.
E13. The blood analysis method according to any one of E10 to E12, wherein in the step of obtaining the fluorescence signal and the scattered light signal, fluorescence, forward scattered light and side scattered light that are produced by cells in the measurement sample are received when the measurement sample is irradiated with light to obtain the fluorescence signal relating to the received fluorescence and the scattered light signal containing a forward scattered light signal relating to the received forward scattered light and a side scattered light signal relating to the received side scattered light, and the blood analysis method further comprises a step of detecting blasts distinguishably from abnormal lymphocytes and atypical lymphocytes based on at least two signals selected from the forward scattered light signal, the side scattered light signal, and the fluorescence signal.
E14. The blood analysis method according to E13, wherein in the step of detecting blasts, cells showing forward scattered light intensity and side scattered light intensity in a predetermined range are detected as blasts distinguishably from abnormal lymphocytes and atypical lymphocytes based on the forward scattering light signal and the side scattering light signal.
E15. The blood analysis method according to any one of E10 to E14, wherein in the step of preparing the measurement sample, the measurement sample is prepared by mixing the hemolyzing agent not substantially containing a cationic surfactant, but containing a nonionic surfactant and an anionic surfactant, the blood specimen, and the fluorescent dye.
E16. A blood analysis method comprising steps of preparing a first measurement sample from a blood specimen and a first reagent; irradiating the first measurement sample prepared with light; receiving fluorescence and scattered light that are produced by cells in the first measurement sample when the first measurement sample is irradiated with light to obtain a first fluorescence signal relating to the received fluorescence and a first scattered light signal relating to the received scattered light; preparing a second measurement sample from a blood specimen and a second reagent; irradiating the second measurement sample prepared with light; receiving fluorescence and scattered light that are produced by cells in the second measurement sample when the second measurement sample is irradiated with light to obtain a second fluorescence signal relating to the received fluorescence and a second scattered light signal relating to the received scattered light; performing a detection process for detecting nucleated erythrocytes in the second measurement sample based on the second fluorescence signal and the second scattered light signal, and determining whether a result of the detection process satisfies a predetermined condition; detecting cells showing fluorescence intensity and scattered light intensity in a predetermined range as abnormal lymphocytes based on the first fluorescence signal and the first scattered light signal if the result of the detection process satisfies the predetermined condition; and performing output based on a result of a detection.
E17. A computer program for enabling a computer including a communication portion and an output portion to analyze a blood specimen, the computer program enabling the computer to perform steps of the communication portion receiving a fluorescence signal and a scattered light signal relating to fluorescence and scattered light that are produced by cells in a measurement sample prepared by mixing a hemolyzing agent not substantially containing a cationic surfactant, but containing a nonionic surfactant, a blood specimen, and a fluorescence dye for staining nucleic acid when the measurement sample is irradiated with light; detecting cells showing fluorescence intensity and scattered light intensity in a predetermined range as abnormal lymphocytes based on the fluorescence signal and the scattered light signal; and enabling the output portion to produce an output based on a result of a detection.
E18. The computer program according to E17, which enables the computer to detect the cells showing fluorescence intensity and scattered light intensity in the predetermined range as abnormal lymphocytes distinguishably from blasts and atypical lymphocytes.
E19. A computer program for enabling a computer including a communication portion and an output portion to analyze a blood specimen, the computer program enabling the computer to perform steps of the communication portion receiving a first fluorescence signal and a first scattered light signal relating to fluorescence and scattered light that are produced by cells in a first measurement sample prepared from a blood specimen and a first reagent when the first measurement sample is irradiated with light, and a second fluorescence signal and a second scattered light signal relating to fluorescence and scattered light that are produced by cells in a second measurement sample prepared from a blood specimen and a second reagent when the second measurement sample is irradiated with light; performing a detection process for detecting nucleated erythrocytes in the second measurement sample based on the second fluorescence signal and the second scattered light signal, and determining whether a result of the detection process satisfies a predetermined condition; detecting cells showing fluorescence intensity and scattered light intensity in a predetermined range as abnormal lymphocytes based on the first fluorescence signal and the first scattered light signal if the result of the detection process satisfies the predetermined condition; and enabling the output portion to perform output based on a result of a detection.

### List of Reference Numerals

- 1: Blood analyzer
- 2: Measurement unit
- 22: Sample preparation portion
- 221a, 221b, 223: Reagent container
- 23: Detection portion
- 231: Flow cell
- 237: Semiconductor laser
- 243: Photodiode
- 246: Photodiode
- 248: Avalanche photodiode
- 5: Information processing unit
- 5a: Computer
- 51: Body
- 51a: CPU
- 51b: ROM
- 51c: RAM
- 51d: Hard disk
- 51h: Image output interface
- 52: Image display portion
- 54a: Computer program
- MC: Mixing chamber
- H: Heater
- D: Optical detector
- R1: Analysis result screen

## Claims

1. A blood analyzer comprising:
a sample preparation portion configured to prepare a first measurement sample from a blood specimen and a first reagent and second measurement sample from a blood specimen and a second reagent;
a light source configured to irradiate the first measurement sample and the second measurement sample with light;
a light receiving portion configured to receive fluorescence and scattered light from cells in the first measurement sample which is irradiated with light by the light source to output a first fluorescence signal relating to the received fluorescence and a first scattered light signal relating to the received scattered light, and configured to receive fluorescence and scattered light from cells in the second measurement sample which is irradiated with light by the light source to output a second fluorescence signal relating to the received fluorescence and a second scattered light signal relating to the received scattered light; and
a cell analysis portion configured to perform a first detection process to detect abnormal lymphocytes based on the first fluorescence signal and the first scattered light signal, and a second detection process to detect nucleated erythrocytes based on the second fluorescence signal and the second scattered light signal;
wherein the cell analysis portion is configured to perform a determination process for determining whether abnormal lymphocytes are present in a blood specimen based on the results of the first detection process and the second detection process.

2. The blood analyzer according to claim 1, further comprising an output portion configured to output the detection results by the cell analysis portion,
wherein the cell analysis portion is configured to control the output portion to output information indicating the possibility that abnormal lymphocytes are present based on the results of the determination process.

3. The blood analyzer according to claim 2, wherein
the cell analysis portion is configured to:
obtain a value reflecting the number of the abnormal lymphocytes based on the first fluorescence signal and the first scattered light signal and compare the obtained value and a first threshold, and
obtain a value reflecting the number of the nucleated erythrocytes based on the second fluorescence signal and the second scattered light signal and compares the obtained value and a second threshold.

4. The blood analyzer according to claim 3, wherein
in the case that the value reflecting the number of the abnormal lymphocytes is greater than the first threshold and the value reflecting the number of the nucleated erythrocytes is smaller than the second threshold, the cell analysis portion controls the output portion to output information indicating the possibility that abnormal lymphocytes are present.

5. The blood analyzer according to claim 3, wherein
in the case that the value reflecting the number of the abnormal lymphocytes is greater than the first threshold and the value reflecting the number of the nucleated erythrocytes is greater than the second threshold, the cell analysis portion controls the output portion to output information indicating the possibility that nucleated erythrocytes are present.

6. The blood analyzer according to claim 3, wherein
in the case that the value reflecting the number of the abnormal lymphocytes is greater than the first threshold and the value reflecting the number of the nucleated erythrocytes is greater than the second threshold, the cell analysis portion controls the output portion not to output information indicating the possibility that abnormal lymphocytes are present.

7. The blood analyzer according to claim 1, wherein
the first reagent is a hemolyzing agent to distinguishably detect abnormal lymphocytes, atypical lymphocytes and blasts.

8. The blood analyzer according to claim 1, wherein
the first reagent contains a nonionic surfactant and does not substantially contain a cationic surfactant.

9. The blood analyzer according to claim 1, wherein
the second reagent is a hemolyzing agent to detect nucleated erythrocytes.

10. The blood analyzer according to claim 1, wherein
the cell analysis portion is configured to detect cells in an area with a fluorescence intensity higher than that in the area where lymphocytes and monocytes appear, as abnormal lymphocytes cells.

11. A blood analysis method comprising:
preparing a first measurement sample from a blood specimen and a first reagent;
irradiating the first measurement sample with light;
receiving fluorescence and scattered light that are produced from cells in the first measurement sample which is irradiated with light to obtain a first fluorescence signal relating to the received fluorescence and a first scattered light signal relating to the received scattered light;
preparing a second measurement sample from a blood specimen and a second reagent;
irradiating the second measurement sample with light;
receiving fluorescence and scattered light that are produced from cells in the second measurement sample which is irradiated with light to obtain a second fluorescence signal relating to the received fluorescence and a second scattered light signal relating to the received scattered light;
detecting abnormal lymphocytes based on the first fluorescence signal and the first scattered light signal;
detecting nucleated erythrocytes based on the second fluorescence signal and the second scattered light signal; and
determining whether abnormal lymphocytes are present in a blood specimen based on the detection results of the detecting nucleated erythrocytes and detecting nucleated erythrocytes.

12. The blood analysis method according to claim 11, further comprising:
outputting information indicating the possibility that abnormal lymphocytes are present based on the results of the determining process.

13. The blood analysis method according to claim 12, further comprising:
obtaining a value reflecting the number of the abnormal lymphocytes based on the first fluorescence signal and the first scattered light signal;
comparing the obtained value and a first threshold;
obtaining a value reflecting the number of the nucleated erythrocytes based on the second fluorescence signal and the second scattered light signal; and
comparing the obtained value and a second threshold.

14. The blood analysis method according to claim 13, further comprising:
in the case that the value reflecting the number of the abnormal lymphocytes is greater than the first threshold and the value reflecting the number of the nucleated erythrocytes is smaller than the second threshold, outputting information indicating the possibility that abnormal lymphocytes are present.

15. The blood analysis method according to claim 13, further comprising;
in the case that the value reflecting the number of the abnormal lymphocytes is greater than the first threshold and the value reflecting the number of the nucleated erythrocytes is greater than the second threshold, outputting information indicating the possibility that nucleated erythrocytes are present.
